# FASCICULE DE BREVET EUROPEEN

(11) **EP 1 323 412 B1**
(45) Date de publication et mention de la délivrance du brevet: **19.10.2016**
(21) Numéro de dépôt: 02292838.6
(22) Date de dépôt: 14.11.2002
(51) Int. Cl.: A61K 8/35, A61K 8/44, A61K 8/49, A61Q 17/04

(54) **Composition filtrante contenant un filtre du type dérivé du dibenzoylméthane et un dérivé de 2-hydroxybenzophénone aminosubstitué**
Ein Lichtschutzmittel vom Dibenzoylmethan-Typ und ein amino-substituiertes 2-Hydroxybenzophenon-Derivat enthaltende Lichtschutzmittelzusammensetzung
Light screening composition comprising a dibenzoylmethane type screening agent and an amino substituted 2-hydroxybenzophenone derivative

(30) Priorité: 07.12.2001 FR 0115858
(43) Date de publication de la demande: 02.07.2003
(73) Titulaire: L'Oréal, 75008 Paris (FR)
(72) Inventeur: Candau, Didier, 91570 Bièvres (FR)
(74) Mandataire: Casalonga

(56) Documents cités:
- EP-A- 1 046 391
- EP-A- 1 133 980
- EP-A1- 0 310 239
- EP-A1- 1 291 007
- EP-A1- 1 291 999
- EP-A1- 1 310 236
- EP-A2- 1 291 009
- EP-A2- 1 310 235
- EP-A2- 1 310 237
- WO-A1-03/039502
- WO-A1-03/039507
- WO-A2-03/039506
- DE-A1- 10 155 716
- DE-A1- 10 155 865
- DE-A1- 10 157 484
- DE-A1- 10 157 489
- DE-A1- 10 157 490
- DE-B- 1 155 900

## Description

L'invention se rapporte à une composition cosmétique ou dermatologique, à usage topique, caractérisée par le fait qu'elle comprend au moins, dans un support cosmétiquement acceptable :
(a) le 4-(ter-butyl)4'-méthoxydibenzoylméthane et
(b) le 2-(4-diéthylamino-2-hydroxybenzoyl)-benzoate de n-hexyle ;
ladite composition ne contenant pas de p-méthylbenzylidènecamphre ; le rapport en poids du dérivé de 2-hydroxybenzophénone sur le dérivé de dibenzoylméthane étant supérieur à 1, et le dérivé de dibenzoylméthane étant présent à des teneurs allant de 2 % à 15 % en poids, par rapport au poids total de la composition.

L'invention se rapporte également à un procédé pour améliorer la stabilisé vis-à-vis du rayonnement UV d'au moins un dérivé du dibenzoylméthane présent dans une composition cosmétique ou dermatolologique ne contenant de p-méthylbenzylidènecamphre consistant à associer audit dérivé de dibenzoylméthane une quantité efficace d'au moins un dérivé de 2-hydroxybenzophénone aminosubstitué particulier, le rapport en poids du dérivé de 2-hydrobenzophénone sur le dérivé de dibenzoylméthane étant supérieur à 1.

Se référant aux demandes de brevet DE 10155865, DE10157484 et DE 10157489, le demandeur a de sa propre initiative limité la portée de la présente demande et présenté des revendications séparées pour l'Allemagne.

On sait que les radiations lumineuses de longueurs, d'onde comprises entre 280 nm et 400 nm permettent le brunissement de l'épiderme humain, et que les rayons de longueurs d'onde plus particulièrement comprises entre 280 et 320 nm, connus sous la dénomination UV-B, provoquent des érythèmes et des brûlures cutanées qui peuvent nuire au développement du bronzage naturel. Pour ces raisons ainsi que pour des raisons esthétiques, il existe une demande constante de moyens de contrôle de ce bronzage naturel en vue de contrôler ainsi la couleur de la peau ; il convient donc de filtrer ce rayonnement UV-B:

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement scolaire. Les rayons UV-A provoquent en particulier une perte d'élasticité de la peau et l'apparition de rides conduisant à un vieillissement cutané prématuré, Ils favorisent le déclenchement de la réaction érythémateuse ou amplifient cette réaction chez certains sujets et peuvent même être à l'origine de réactions phototoxiques ou photo-allergiques. Ainsi, pour des raisons esthétiques et cosmétiques telles que la conservation de l'élasticité naturelle de la peau par exemple, de plus en plus de gens désirent contrôler l'effet des rayons UV-A sur leur peau. Il est donc souhaitable de filtrer aussi le rayonnement UV-A.

A cet égard, une famille de filtres UV-A particulièrement intéressante est actuellement constituée par les dérivés du dibenzoylméthane, et notamment le 4-ter-butyl-4'-méthaxydibenzoyl méthane, qui présentent en effet un fort pouvoir d'absorption intrinsèque. Ces dérivés du dibenzoylméthane, qui sont maintenant des produits bien connus en soi à titre de filtres actifs dans les UV-A, sont notamment décrits dans les demandes de brevets français FR-A-2326405 et FR-A-2440933, ainsi que dans la demande de brevet européen EP-A-0114607 ; le 4-ter-butyl-4'-méthoxydibenzoyl méthane est par ailleurs actuellement proposé à la vente sous la dénomination commerciale de "PARSOL 1789" par la Société HOFFMANN LAROCHE.

Malheureusement, il se trouve que les dérivés du dibenzoylméfhane sont des produits relativement sensibles au rayonnement ultraviolet (surtout UV-A), c'est-à-dire, plus précisément, qu'ils présentent une fâcheuse tendance à se dégrader plus ou moins rapidement sous l'action de ce dernier. Ainsi, ce manque substantiel de stabilisé photochimique des dérivés du dibenzoylméthane face au rayonnement ultraviolet auquel ils sont par nature destinés à être soumis, ne permet pas de garantir une protection constante durant une exposition solaire prolongée, de sorte que des applications répétées à intervalles de temps réguliers et rapprochés doivent être effectuées par l'utilisateur pour obtenir une protection efficace de la peau contre les rayons UV.

La photostabilisation des dérivés du dibenzoylméthane vis-à-vis du rayonnement UV constitue, à ce jour, un problème qui n'a pas encore été résolu de manière complètement satisfaisante.

On connaît dans les demandes de brevet DE 100 12 408 et EP1046391 des compositions solaires de dérivés de 2-hydroxybenzophénone aminosubstitués pouvant contenir d'autres filtres complémentaires comme les dérivés de dibenzoylméthane en présence du p-méthylbenzylidènecamphre.

Le document EP 1291009 divulgue en particulier une composition cosmétique (ex. 12) exclue de la portée de l'invention au moyen d'un disclaimer (composition 6 de la revendication 1).
Le document EP 1448157 divulgue en particulier deux compositions cosmétiques (ex. 11.3 et 13.3) exclues de la portée de l'invention au moyen de deux disclaimers (compositions 9 et 12 de la revendication 1).

Le document EP 1446094 divulgue en particulier une composition cosmétique (ex. 1.6) exclue de la portée de l'invention au moyen d'un disclaimer (composition 15 de la revendication 1).

Le document EP 1480607 divulgue en particulier une composition cosmétique (ex. 1.6) exclue de la portée de l'invention au moyen d'un disclaimer (composition 24 de la revendication 1).

Le document EP 1310237 divulgue en particulier une composition cosmétique (ex. 1.6) exclue de la portée de l'invention au moyen d'un disclaimer (composition 37 de la revendication 1).

Le document EP 1310239 divulgue en particulier deux compositions cosmétiques (ex. 1.1 et 3.3) exclues de la portée de l'invention au moyen de deux disclaimers (compositions 44 et 47 de la revendication 1).

Or, la Demanderesse vient maintenant de découvrir, de façon surprenante, qu'en associant aux dérivés du dibenzoylméthane mentionnés ci-dessus une quantité d'au moins un dérivé de 2-hydroxybenzophénone aminosubstitué particulier, il était possible d'améliorer de manière substantielle et remarquable, la stabilité photochimique (ou photostabilite) de ces mêmes dérivés du dibenzoylméthane.

Ainsi, conformément à l'un des objets de la présente invention, il est maintenant proposé une nouvelle composition cosmétique ou dermatologique, à usage topique, caractérisée par le fait qu'elle comprend au moins, dans un support cosmétiquement acceptable :
(a) le 4-(ter-butyl)4'-méthoxy dibenzoyimôthane et
(b) le 2-(4-diéthylamino-2-hydroxybenzoyl)-benzoate de n-hexyle ; ladite composition ne contenant pas de p-méthylbenzylidène camphre ; le rapport en poids du dérivé de 2-hydroxybenzophénone sur le dérivé de dibenzoylméthane étant supérieur à 1, et le dérivé de dibenzoylméthane étant présent à des teneurs allant de 2 % à 15 % en poids, par rapport au poids total de la composition ; sous réserve que la composition soit différente des formulations suivantes dans lesquelles les quantités sont exprimées en pourcentage en poids par rapport au poids total de la composition :

| **Ingrédients** | **Composition 6** |
|---|---|
| Cetyl Dimethicone Copolyol | 0,5 |
| Polyricinoléate de polyglycérol | 5,0 |
| 2-(4-diéthylamino-2-hydroxybenzoyl)-benzoate de n-hexyle | 5,0 |
| Phénylbenzimidazole, acide sulfonique | 2,5 |
| Octocrylène | 5,0 |
| Diméthicone-diéthylbehzylmalonate | 4,0 |
| Homosalate | 4,0 |
| Butyl méthoxydibenzoylméthane | 3,0 |
| Oxyde de Zinc micronisé | 5,0 |
| Coco-caprylate/caprate | 10,0 |
| Diméthicone | 2,5 |
| Polydécène | 7,0 |
| Polyisobutène | 2,0 |
| Chlorure de sodium | 0,45 |
| Parfum | 0,15 |
| Glycine soja | 2,0 |
| Phénoxyéthanol | 1,0 |
| Eau | qsp 100 |

| **Ingrédients** | **Composition 9** |
|---|---|
| Monostéarate de glycérol autoémulsionnant | 3,00 |
| Polyoxyéthylène(30)cétylstéaryléther | 1,00 |
| Alcool stéarylique | 3,00 |
| Butyl Méthoxydibenzoylméthane | 2,00 |
| Ethylhexyl Triazone | 3,00 |
| 2-(4-diéthylamino-2-hydroxybenzoyl)-benzoate de n-hexyle | 3,50 |
| Acide phénylèn-1,4-bis(monosodium)-2-benzimidazolyl-5,7-disulfonique | 0,5 |
| Oxyde de dioctyle | 3,50 |
| Dicaprylyl Carbonate | 6,00 |
| Diméthicone Polydiméthylsiloxane | 1,00 |
| Glycérine | 5,00 |
| Acétate de tocophéryle | 0,25 |
| Acide dioique | 0,20 |
| Diéthylhexyl-2,6-naphthalate | 2,00 |
| Alpha-Glucosylrutin | 0,20 |
| Paraben | 0,50 |
| Konkaben LMB® | 0,40 |
| Ethanol | 1,50 |
| Parfum | qs |
| Eau | qsp 100 |

| **Ingrédients** | **Composition 12** |
|---|---|
| PEG-30-dipolyhydroxystéarate | 5,00 |
| Butyl méthoxydibenzoylméthane | 2,00 |
| Ethylhexyl Triazone | 3,00 |
| 2-(4-diéthylamino-2-hydroxybenzoyl)-benzoate de n-hexyle | 4,50 |
| Octocrylène | 4,00 |
| Acide phénylèn-1,4-bis(monosodium)-2-benzimidazolyl-5,7-disulfonique | 0,50 |
| Diméthicone-diéthylbenzylmalonate | 5,50 |
| Dioxyde de Titane | 1,50 |
| Oxyde de Zinc | 2,00 |
| Huile de paraffine | 10,0 |
| Esters d'acides caprylique et caprique et de butylène glycol | 2,00 |
| Dicaprylyl Carbonate | 6,00 |
| Diméthicone Polydiméthylsiloxane | 1,00 |
| Beurre de karité | 3,00 |
| Diéthylhexyl-2,6-naphthalate | 6,50 |
| Octoxyglycérine | 1,00 |
| Glycine Soja | 1,50 |
| Chlorure de magnésium | 1,00 |
| Acétate de tocophéryle | 0,25 |
| Acide Dioique | 0,50 |
| Paraben | 0,50 |
| Konkaben LMB® | 0,40 |
| Ethanol | 1,50 |
| Parfum | qs |
| Eau | qsp 100 |

| **Ingrédients** | **Composition 15** |
|---|---|
| Monostérate de glycérine SE | 1,50 |
| PEG-100 stéarate | 2,00 |
| Cetearyl sulfate | 0,75 |
| Alcool stéarylique | 2,00 |
| 2-(4-diéthylamino-2-hydroxybénzoyl)-benzoate de n-hexyle | 4,50 |
| Ethylhexyl Triazone | 2,00 |
| Drométrizole Trisiloxane | 1,00 |
| Butyl méthoxydibenzoylméthane | 2,00 |
| Acide téréphtalylidène dicamphre sulfonique | 0,50 |
| Oxyde de Zinc HP1® | 2,00 |
| C₁₂-C₁₅ Alkyl Benzoate | 7,00 |
| Dicaprylylcarbonate | 2,00 |
| Glycine Soja | 1,50 |
| Phénoxyéthanol | 0,40 |
| Ethanol | 4,50 |
| Parfum | 0,40 |
| Eau | qsp 100 |

| **Ingrédients** | **Composition 24** |
|---|---|
| Monostérate de glycérine SE | 1,50 |
| PEG-40 stéarate | 2,00 |
| Cetearylsulfate | 0,75 |
| Alcool stéarylique | 2,00 |
| 2-(4-diéthylamino-2-hydroxybenzoyl)-benzoate de n-hexyle | 4,50 |
| Butyl méthoxydibenzoylméthane | 2,00 |
| Ethyhexyl Triazone | 2,00 |
| Drométrizole Trisiloxane | 1,00 |
| Acide téréphtalylidène dicamphre sulfonique | 0,50 |
| Diéthylhexyl-2,6-naphthalate | 5,50 |
| Oxyde de Zinc HP1® | 2,00 |
| C₁₂-C₁₅Alky) Benzoate | 7,00 |
| Dicaprylylcarbonate | 2,00 |
| Glycine Soja | 1,50 |
| Phénoxyéthanol | 0,40 |
| Ethanol | 4,50 |
| Parfum | 0,40 |
| Eau | qsp 100 |

| **Ingrédients** | **Composition 37** |
|---|---|
| Glycéryl Stéarate Citrate | 1,00 |
| PEG-100 stéarate | 2,00 |
| Cétylphosphate | 0,75 |
| Alcool stéarylique | 2,00 |
| Dermacryl 79® | 2,00 |
| 2-(4-diéthylamino-2-hydroxybenzoyl)-benzoate de n-hexyle | 4,50 |
| Butyl méthoxydibenzoyiméthane | 2,00 |
| Ethyhexyl Triazone | 2,00 |
| Drométrizole Trisiloxane | 1,00 |
| Acide téréphtalylidène dicamphre sulfonique | 0,50 |
| Diéthylhexyl-2,6-naphthalate | 10,00 |
| Oxyde de Zinc HP1® | 2,00 |
| C₁₂-C₁₅ Alkyl Benzoate | 7,00 |
| Dicaprylylcarbonate | 2,00 |
| Glycine Soja | 1,50 |
| Phénoxyéthanol | 0,40 |
| Ethanol | 4,50 |
| Parfum | 0,40 |
| Eau | Qsp 100 |

| **Ingrédients** | **Composition 44** |
|---|---|
| Monostérate de glycérine SE | 0,50 |
| Glycéryl Stéarate Citrate | 2,00 |
| PEG-40 Stéarate | 0,50 |
| 2-(4-diéthylamino-2-hydroxybenzoyl)-benzoate de n-hexyle | 4,00 |
| Butyl méthoxydibenzoylméthane | 2,00 |
| Ethylhexyl triazone | 4,00 |
| Parsol SLX® | 3,50 |
| Mexoryl SX® | 0,25 |
| Bisimidazylate | 1,00 |
| Phénylbenzimidazole, acide sulfonique | 0,50 |
| Dioxyde de Titane MT100 TV® | 1,00 |
| Esters d'acides caprylique et caprique et de butylène glycol | 5,00 |
| Cyclométhicone | 2,00 |
| PVP/Eicosène Copolymer | 0,50 |
| Glycérine | 3,00 |
| Propylène Glycol | 1,50 |
| Gomme de Xanthane | 0,15 |
| Acétate de vitamine E | 0,50 |
| Alpha-glucosylrutine | 0,35 |
| Glycine Soja | 1,00 |
| Pentasodium Ethylèndiamintétraméthylphosphonate (PETP) | 0,25 |
| EDTA Trisodium | 0,10 |
| Méthylparabène | 0,15 |
| Phénoxyéthanol | 1,00 |
| Parfum | 0,20 |
| Eau | qsp 100 |

| **Ingrédients** | **Composition 47** |
|---|---|
| Lauryl Diméthicone Copolyol | 1,00 |
| PEG-30-dipolyhydroxystéarate | 5,00 |
| 2-(4-diéthyiamino-2-hydroxybenzoyl)-benzoate de n-hexyle | 5,00 |
| Méthylène Bis-Benzotriazolyl Tétraméthylbutylphénol | 2,00 |
| Butyl méthoxy-dibenzoylméthane | 2,00 |
| Ethylhexyl Triazone | 3,00 |
| Parsol SLX® | 3,50 |
| Octocrylène | 8,00 |
| Bisimidazylate | 0,50 |
| Dioxyde de Titane MT-100 Z | 3,00 |
| Oxyde de Zinc Z-Cote HP1® | 6,00 |
| Huile minérale | 10,00 |
| Esters d'acides caprylique et caprique et de butylène glycol | 2,00 |
| Cyclométhicone | 5,00 |
| Pentasodium Ethylèndiamintétraméthylphosphonate (PETP) | 0,25 |
| Ethylhexyloxyglycérine | 1,00 |
| Glycine Soja | 1,50 |
| Butylène glycol | 10,00 |
| MgCl₂ | 1,00 |
| Vitamine E | 0,25 |
| Ethanol | 4,50 |
| Parfum | 0,20 |
| Eau | qsp 100 |

L'invention se rapporte également à un procédé pour améliorer la stabilité vis-à-vis du rayonnement UV d'au moins un dérivé du dibenzoylméthane dans une composition cosmétique ou dermatolologique ne contenant de p-méthylbenzylidènecamphre consistant à associer audit dérivé de dibenzoylméthane une quantité efficace d'au moins un dérivé de 2-hydroxybenzophénone aminosubstitué tel que décrit ci-dessus, le rapport en poids du dérivé de 2-hydrcxybenzophénone sur le dérivé de dibenzoylméthane étant supérieur à 1.

Par quantité efficace de dérivé de 2-hydroxybenzophénone aminosubstitué conforme à l'invention, on entend une quantité suffisante pour obtenir une amélioration notable et significative de la photostabilité du ou des dérivés du dibenzoylméthane de la composition cosmétique photoprotectrice. Cette quantité minimale en agent photostabilisant à mettre en oeuvre, qui peut varier selon la nature du support cosmétiquement acceptable retenu pour la composition, peut être déterminée sans aucune difficulté au moyen d'un test classique de mesure de photostabilité.

D'autres caractéristiques, aspects et avantages de la présente invention apparaîtront à la lecture de la description détaillée qui va suivre.

Le rapport en poids du dérivé de 2-hydroxybenzophénone aminosubstitué sur le dérivé de dibenzoylméthane est supérieur à 1.

Les dérivés de 2-hydroxybenzophénone aminosubstitués répondent à la formule (I) suivante : 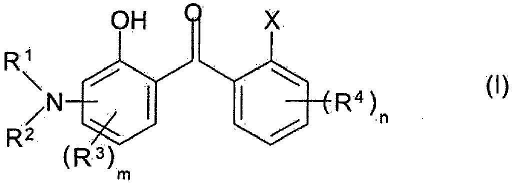 dans laquelle:
R¹ et R² , identiques ou différents, désignent un atome d'hydrogène, un radical alkyle en C₁-C₂₀, un radical alcènyle en C₂-C₁₀, un radical cycloalkyle en C₃-C₁₀, un radical cycloalcènyle en C₃-C₁₀ ;
R¹ et R² peuvent également former avec l'atome d'azote avec lequel ils sont liés un cycle à 5 ou 6 chaînons ;
R³ et R⁴, identiques ou différents, désignent un radical alkyle en C₁-C₂₀, une radical alcènyle en C₂-C₁₀, un radical cycloalkyle en C₃-C₁₀, un. radical cycloalcènyle en C₃-C₁₀, un radical alcoxy en C₁-C₁₂, un radical (C₁-C₂₀)alcoxycarbonyle, un radical alkylamino en C₁-C₁₂, un radical dialkylamino en C₁-C₁₂, un radical aryle ou un hétéroaryle éventuellement, substitué, un substituant hydrosolubilisant choisi parmi un groupe carboxylate, un groupe sulfonate ou un reste ammonium ; X désigne un atome d'hydrogène, un groupe COOR⁵ ou CONR⁶R⁷;
R⁵, R⁶ et R⁷, identiques ou différents, désignent un atome d'hydrogène, un radical alkyle en C₁-C₂₀, un radical alcènylé en C₂-C₁₀, un radical cycloalkyle en C₃-C₁₀, un radical cycloalcènyle en C₃-C₁₀, un groupe -(Y_{O})ₒ-Z ou un groupe aryle ;
Y désigne -(CH₂)₂₋, -(CH₂)₃- -(CH₂)₄-, -CH-CH₃-CH₂- ;
Z représente -CH₂-CH₃, -CH₂CH₂CH₃, -CH₂-CH₂-CH₂-CH₃, -CH(CH₃)-CH₃ ;
m est un entier variant de 0 à3 ;
n est un entier variant de 0 à 3 ;
o est un entier variant de 1 à 2.

Comme radicaux alkyle en C₁-C₂₀, on peut citer par exemple : méthyle, éthyle, n-propyle, 1-méthyléthyle, n-butyle, 1-méthylpropyle, 2-méthylpropyle, 1,1-diméthyléthyle, n-pentyle, 1-méthylbutyle, 2-méthylbutyle, 3-méthylbutyle, 2,2-diméthylpropyle, 1-éthylpropyle, n-hexyle, 1,1=diméthylpropyle, 1,2-diméthylpropyle, 1-méthylpentyle, 2-méthylpentyle, 3-méthylpentyle, 4-methylpentyle, 1,1-diméthylbutyle, 1,2-diméthylbutyle, 1,3-diméthylbutyle, 2,2-diméthylbutyle, 2,3-diméthylbutyle, 3,3-diméthylbutyle, 1-éthylbutyle, 2-éthylbutyle, 1,2,2-triméthylpropyle, 1-éthyl-1-méthylpropyle, 1-éthyl-2-méthylpropyle, n-heptyle, n-octyle, n-nonyle, n-décyle, n-undécyle, n-dodécyle, n-tridécyle, n-tétradécyle, n-pentadécyle, n-hexadécyle, n-heptadécyle, n-octadécyle, n-nonadécyle ou n-eicosyle.

Comme groupes alcènyle en C₂-C₁₀, on peut citer par exemple : vinyle, n-propènyle, isopropènyle, 1-butènyle; 2-butènyle, 1-pentènyle, 2-pentènyle, 2-méthyl-1-butènyle, 2-méthyl-2-butènyle, 3-méthyl-1-butènyle, 1-hexènyle, 2-hexènyle, 1-heptènyle, 2-heptènyle, 1-octènyle, 2-octènyle.

Comme radicaux alcoxy en C₁-C₁₂, on peut citer : méthoxy, éthoxy, n-propoxy, n-butoxy, n-pentoxy, 1-méthylpropoxy, 3-méthylbutoxy, 2,2-diméthylpropoxy, 1-méthyl- 1-éthylpropoxy, octoxy, 2-méthylpropoxy, 1,1-diméthylpropoxy, hexoxy, heptoxy, 2-éthylhexoxy.

Comme radicaux cycloalkyles en C₃-C₁₀, on peut citer par exemple : cyclopropyle, cyclobutyle, cyclopentyle, çyclohexyle, cycloheptyle, 1-méthylcyclopropyle, 1-éthylcyclopropyle, 1-propylyclopropyle, 1-butylcyclopropyle, 1-pentylcyclopropyle, 1-méthyl-1-butylcyclopropyle, 1,2-diméthylcyclypropyle, 1-méthyl-2-éthyEcyclopropyle, cyclooctyle, cyclononyle ou cyclodécyle.

Comme radicaux cycloalcènyles en C₃-C₁₀ ayant une ou plusieurs doubles liaisons, on peut citer : cyclopropènyle, cyclobutènyle, cyclopentènyle, cyclopentadiènyle, cyclohexènyle, 1,3-cyclohexadiènyle, 1,4-cyclohexadiènyle, cycloheptènyle, cycloheptatriènyle, cyclooctènyle, 1,5-cyclooctadiènyle, cyclooctatétraènyle, cyclononènyle ou cyclodécènyle.

Les radicaux cycloalkyles ou cycloalcényles peuvent comporter un ou plusieurs substituants (de préférence de 1 à 3) chois par exemple parmi halogène comme chlore, fluor ou brome ; cyano ; nitro ; amino C₁-C₄-alkylamino ; C₁-C₄ dialkylamino ; C₁-C₄alkyle ; C₁-C₄-alcoxy ; hydroxy ; ils peuvent également comporter de 1 à 3 hétéroatomes comme souffre, oxygène ou azote dont les valences libres peuvent être occupées par un hydrogène ou un radical allyle en C₁-C₄.

Les groupes aryles sont de préférence choisis parmi les cycles phényle ou naphtyle, lesquels pouvant comporter un ou plusieurs substituants (de préférence de 1 à 3) chois par exemple parmi halogène comme chlore, fluor ou brome ; cyano ; nitro. ; amine ; C₁-C₄-alkylamino ; C₁-₄ dialkylamino ; C₁-C₄alkyle ; C₁-C₄-alcoxy ; hydroxy. On préfère plus particulièrement phényle, méthoxyphényle et naphtyle.

Les groupes hétéroaryles comportent en général un ou plusieurs hétéroatomes choisis parmi souffre, oxygène ou azote.

Les groupes hydrosolubilisants sont par exemple des groupes carboxylates, sulfonates et plus particulièrement leurs sels avec des cations physiologiquement acceptables comme les sels de métaux alcalins ou les sels de trialkylammonium comme les sels de tri(hydroxyalkyl)ammonium ou de 2-méthylpropan-1-ol-2-ammonium. On peut également citer les groupes ammonium comme les alkylammoniums et leurs formes salifiées avec des anions physiologiquement acceptables.

Comme exemples de cycle à 5 ou 6 chaînons formé par les radicaux R¹ et R² avec l'atome d'azote, on peut citer en particulier pyrrolidine ou pipéridine.

Les groupes amino peuvent être fixés sur le noyau benzénique en position ortho, méta ou para par rapport au radical carbonyle et plus préférentiellement en para.

Une famille de composés de formule (I) comprend ceux choisis parmi ceux de formule (Ia) suivante ; 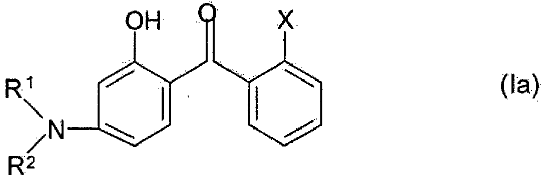 dans laquelle :
R¹et R² , identiques ou différents, désignent un atome d'hydrogène, un radical alkyle en C₁-C₁₂ ou forment avec l'atome d'azote avec lequel ils sont liés un cycle à 5 ou 6 chaînons ;
X désigne COOR⁵ ou CONR⁶R⁷;
R⁵ désigne un atome d'hydrogène, un radical alkyle en C₁-C₁₂, un radical cycloalkyle en C₃-C6.
R⁶ et R , identiques ou différents, désignent un atome d'hydrogéne, un radical alkyle en C₁-C₁₂, un radical cycloalkyle en C₅-C₆.

Des composés de formule (Ia) sont ceux pour lesquels :
R¹ et R², identiques ou différents, désignent un radical alkyle en C₁-C₄ et plus particulièrement éthyle ;
R⁵ désigne un radical allyle en C₃-C₈,
R⁶ et R , identiques ou différents, désignent un radical alkyle en C₁-C₈,

Une autre famille de composés de formule (I) comprend ceux choisis parmi ceux de formule (Ib) suivante : 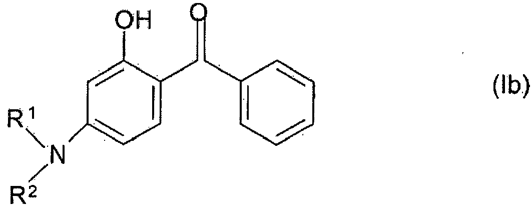 dans laquelle :
R¹ et R², identiques ou différents, désignent un radical alkyle en C₁-C₁₂ ou forment avec l'atome d'azote avec lequel ils sont liés un cycle à 5 ou 6 chaînons.

Parmi les composés de formule (Ib), on peut citer plus particulièrement :
- le (4-diéthylamino-2-hydroxyphényl)-phénylcétone.
- le (4-pyrrolidino-2-hydroxyphényl)-phénylcétone..

Une famille de composés de formule (I) comprend ceux choisis parmi ceux de formule (Ic) suivante : 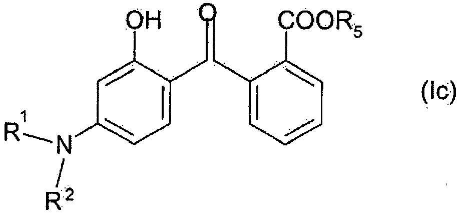 dans laquelle :
R¹ et R , identiques ou différents, désignent un atome d'hydrogène, un radical alkyle en C₁-C₈ ou forment avec l'atome d'azote avec lequel ils sont liés un cycle à 5 ou 6 chaînons ;
R⁵ désigne un atome d'hydrogène, un radical allyle en C₁-C₁₂, un radical cycloalkyle en C₃-C₆

Parmi les composés de formule (Ic), on peut citer :
- le 2-(4-pyrrolidino-2-hydroxybenzoyl)-benzoate
- le 2-(4-diéthylamino-2-hydroxybenzoyl)-benzoate de méthyle
- le 2-(4-diéthylamino-2-hydroxybenzoyl)-benzoate de 2-éthylhexyle
- le 2-(4-diéthylamino-2-hydroxybenzoyl)-benzoate de cyclohexyle
- le 2-(4-diéthylamino-2-hydroxybenzoyl)-benzoate de n-hexyle
- le 2-(4-dibutylamino-2-hydroxybenzoyl)-benzoate
- le 2-(4-dibutylamino-2-hydroxybenzoyl)-benzoate de méthyle
- le 2-(4-dibutylamino-2-hydroxybenzoyl)-benzoate d'isobutyle.

Dans le cadre de la présente invention, on utilise le 2-hydroxybenzoyl)-benzoate de n-hexyle.

Les composés de formule (I) tels que définis ci-dessus sont connus en eux-mêmes et leurs structures et leurs synthèses sont décrites dans les demandes de brevet EP-A-1046391 et DE100 12 408 (faisant partie intégrante du contenu de la description).

Les dérivés de 2-hydroxybenzophénone aminosubstitués sont présents de préférence dans la composition de l'invention dans des proportions allant plus préférentiellement de 0,1 à 15% en poids et plus préférentiellement de 1 à 10% en poids et plus particulièrement de 2 à 8% en poids par rapport au poids total de la composition.

Comme indiqué précédemment, les dérivés du dibenzoylméthane destinés à être photostabilisés dans le cadre de la présente invention sont des produits déjà bien connus en soi et décrits notamment dans les documents FR2326405, FR2440933 et EP0114607 précités, documents dont les enseignements sont, pour ce qui touche à la définition même de ces produits, totalement inclus à titre de références dans la présente description.

Selon la présente invention, on peut bien entendu mettre en oeuvre un ou plusieurs dérivés du dibenzoylméthane. Néanmoins les compositions de l'invention doivent contenir le 4-(ter.-butyl)4-méthoxy dibenzoylméthane de n-hexyle.

Parmi les dérivés du dibenzoylméthane, destinés à être stabilisés, on peut notamment citer, de manière non limitative :
- le 2-méthyldibenzoylméthane
- le 4-méthyldibenzoylméthane
- le 4-isopropyldibenzoylméthane
- le 4-tert.-butyldibenzoylméthane
- le 2,4-diméthyldibenzoylméthane
- le 2,5-diméthyldibenzoylméthane
- le 4,4'-düsopropyldibenzoylméthane
- le 4,4'-diméthoxydibenzoylméthane
- le 4-tert.-butyl-4'-méthoxydibenzoylméthane
- le 2-méthyl-5-isopropyl-4'-méthoxydibenzoylméthane
- le 2-méthyl-5-tert.-butyl-4'-méthoxydibenzoylméthane
- le 2,4-diméthyl-4'-méthoxydibenzoylméthane
- le 2,6-diméthyl-4-tert.-butyl-4'-méthoxydibenzoylméthane

Parmi les dérivés du dibenzoylméthane mentionnés ci-dessus, on préfère tout particulièrement, selon la présente invention, mettre en oeuvre le 4-(ter.-butyl) 4'-méthoxy dibenzoylméthane, notamment celui proposé à la vente sous la dénomination commerciale de "PARSOL 1789" par la Société HOFFMANN LAROCHE, ce filtre répondant donc à la formule développée suivante :

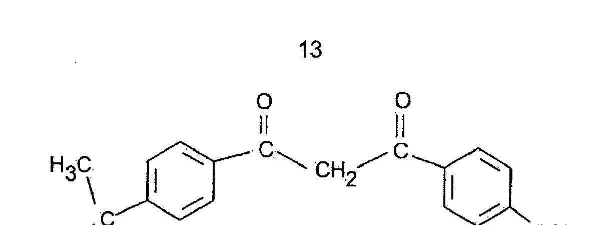

Un autre dérivé du dibenzoytméthane préféré selon le procédé de la présente invention est te 4-isopropyl-dibenzoylméthane, filtre vendu sous la dénomination de "EUSOLEX 8020" par la Société MERCK, et répondant à la formule développée suivante :

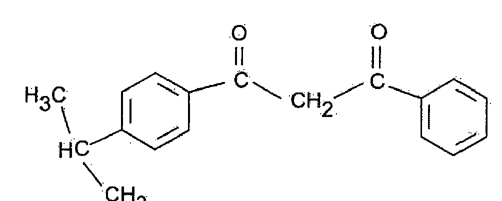

Le ou les dérivés du dibenzoyiméthane à stabiliser sont présents dans les compositions à des teneurs qui varient de préférence de 2 à 15% en poids et plus préférentiellement de 2 à 10% en poids et plus particulièrement de 2 à 8% en poids par rapport au poids total de la composition.

Les compositions conformes à l'invention peuvent comporter en plus d'autres filtres UV organiques complémentaires actifs dans l'UVA et/ou l'UVB (absorbeurs), hydrosolubles ou liposolubles ou bien insolubles dans les solvants cosmétiques couramment utilisés.

Les filtres UV organiques complémentaires sont notamment choisis parmi les anthranilates ; les dérivés salicyliques, les dérivés cinnamiques, les dérivés du camphre autres que le p-méthylbenzylidènecamphre ; les dérivés de triazine tels que ceux décrits dans les demandes de brevet US 4367390, EP863145, EP517104, EP570838, EP796851, EP775698, EP878469 et EP933376 ; les dérivés de la benzophénone autres que ceux de formule (I); les dérivés de β,β-diphénylacrylate, les dérivés de benzotriazole, les dérivés de benzimidazole ; les imidazolines ; les dérivés de l'acide p-aminobenzoïque (PABA) ; les dérivés de méthylène bis-(hydroxyphényl benzotriazole) tels que décrits dans les demandes US5,237,071, US5,166,355, GB2303549, D19726184 et EP893119 ; les polymères filtres et silicones filtres tels que ceux décrits notamment dans la demande WO93/04665 ; les dimères dérivés d'α-alkylstyrène tels que ceux décrits dans la demande de brevet DE19855649 ; les 4,4-diarylbutadiènes tels que décrits dans les demandes EP0967200, DE19746654, DE19755649, EP-A-1008586.

Comme exemples de filtres organiques, on peut citer désignés ci-dessous sous leur nom INCI :
Dérivés de l'acide para-aminobenzoique:
   - PABA,
   - Ethyl PABA,
   - Ethyl Dihydroxypropyl PABA,
   - Ethylhexyl Diméthyl PABA vendu notamment sous le nom « ESCALOL 507 » par ISP,
   - Glyceryl PABA,
   - PEG-25 PABA vendu sous le nom « UVINUL P25 » par BASF,
Dérivés salicyliques :
   - Homosalate vendu sous le nom « EUSOLEX HMS » par RONA/EM INDUSTRIES,
   - Ethylhexyl Salicylate vendu sous le nom « NEO HELIOPAN OS » par HAARMANN et REIMER,
   - Dipropyleneglycol Salicylate vendu sous le nom « DIPSAL » par SCHER,
   - TEA Salicylate, vendu sous le nom « NEO HELIOPAN TS » par HAARMANN et REIMER,
Dérivés cinnamiques :
   - Ethylhexyl Methoxycinnamate vendu notamment sous le nom commercial « PARSOL MCX » par HOFFMANN LA ROCHE,
   - Isopropyl Methoxy cinnamate,
   - Isoamyl Methoxy cinnamate vendu sous le nom commercial « NEO HELIOPAN E 1000 » par HAARMANN et REIMER,
   - Cinoxate,
   - DEA Methoxycinnamate,
   - Diisopropyl Methylcinnamate,
   - Glyceryl Ethylhexanoate Dimethoxycinnamate
Dérivés de β,β'-diphénylacrylate :
   - Octocrylene vendu notamment sous le nom commercial « UVINUL N539 » par BASF,
   - Etocrylene, vendu notamment sous le nom commercial « UVINUL N35 » par BASF,
Dérivés delta benzophenone :
   - Benzophenone-1 vendu sous le nom commercial « UVINUL 400 » par BASF,
   - Benzophenone-2 vendu sous le nom commercial « UVINUL D50 » par BASF
   - Benzophenone-3 ou Oxybenzone, vendu sous le nom commercial « UVINUL M40 » par BASF,
   - Benzophenone-4 vendu sous le nom commercial « UVINUL MS40 » par BASF,
   - Benzophenone-5
   - Benzophenone-6 vendu sous le nom commercial « HELISORB 11 » par NORQUAY
   - Benzophenone-8 vendu sous le nom commercial « SPECTRA-SORB UV-24 » PAR AMERICAN CYANAMID
   - Benzophenone-9 vendu sous le nom commercial« UVINUL DS-49» par BASF,
   - Benzophenone-12
Dérivés du benzylidène camphre :
   - 3-Benzylidene camphor fabriqué sous le nom « MEXORYL SD» par CHIMEX,
   - Benzylidene Camphor Sulfonic Acid fabriqué sous le nom « MEXORYL SL» par CHIMEX,
   - Camphor Benzalkonium Methosulfate fabriqué sous le nom « MEXORYL SO » par CHIMEX,
   - Terephthalylidene Dicamphor Sulfonic Acid fabriqué sous le nom « MEXORYL SX » par CHIMEX,
   - Polyacrylamidomethyl Benzylidene Camphor fabriqué sous le nom « MEXORYL SW » par CHIMEX,
Dérivés du phenyl benzimidazole :
   - Phenylbenzimidazole Sulfonic Acid vendu notamment sous le nom commercial « EUSOLEX 232 » par MERCK,
   - Disodium Phenyl Dibenzimidazole Tetra-sulfonate vendu sous le nom commercial « NEO HELIOPAN AP » par HAARMANN et REIMER,
Dérivés de triazine :
   - Anisotriazine vendu sous le nom commercial « TINOSORB S » par CIBA SPECIALTY CHEMICALS
   - Ethylhexyl triazone vendu notamment sous le nom commercial «UVINUL T150 » par BASF,
   - Diethylhexyl Butamido Triazone vendu sous le nom commercial « UVASORB HEB » par SIGMA 3V,
   - la 2,4,6-tris-(4'-amino benzalmalonate de diisobutyle)-s-triazine.
Dérivés du phenyl benzotriazole :
   - Drometrizole Trisiloxane vendu sous le nom « SILATRIZOLE » par RHODIA CHIMIE,
   - Methylène bis-Benzotriazolyl Tetramethytbutylphénol, vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,
Dérivés anthraniliques :
   - Menthyl anthranilate vendu sous le nom commercial commercial « NEO HELIOPAN MA » par HAARMANN et REIMER,
Dérivés d'imidazolines :
   - Ethylhexyl Dimethoxybenzylidene Dioxoimidazoline Propionate,
Dérivés du benzalmalonate :
   - Polyorganosiloxane à fonctions benzalmalonate vendu sous la dénomination commerciale « PARSOL SLX » par HOFFMANN LA ROCHE
Dérivés de 4,4-diarylbutadiene
   1,1-dicarboxy (2,2'-diméthyl-propyl)-4,4-diphénylbutadiène
      et leurs mélanges.

Les filtres UV organiques plus particulièrement préférés sont choisis parmi les composés suivants :
- Ethylhexyl Salicylate,
- Ethylhexyl Methoxycinnamate
- Octocrylene
- Phenylbenzimidazole Sulfonic Acid,
- Terephthalylidene Dicamphor Sulfonic,.
- Benzophenone-3,
- Benzophenone-4,
- Benzophenone-5,
- 4-Methylbenzylidene camphor,
- Disodium Phenyl Dibenzimidazole Tetra-sulfonate
- Anisotriazine,
- Ethylhexyl triazone,
- Diethylhexyl Butamido Triazone,
- la 2,4,6-tris-(4'-amino benzalmalonate de diisobutyle)-s-triazine
- Methylene bis-Benzotriazolyl Tetramethylbutylphenol,
- Drometrizole Trisiloxane,
- 1,1-dicarboxy (2,2'-diméthyl-propyl)-4,4-diphénylbutadiène.
   et leurs mélanges.

Les compositions cosmétiques selon l'invention peuvent encore contenir des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photo protecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP-A-0518772 et EP-A-0518773.

Les compositions selon l'invention peuvent également contenir des agents de bronzage et/ou de brunissage artificiels de la peau (agents autobronzants), tels que par exemple de la dihydroxyacétone (DHA).

Les compositions de invention peuvent comprendre en outre des adjuvants cosmétiques classiques notamment choisis parmi les corps gras, les solvants organiques, les épaississants ioniques ou non ioniques, les adoucissants, les antioxydants, les agents anti radicaux libres, les opacifiants, les stabilisants, les émollients, les silicones, les α-hydroxyacides, les agents anti-mousse, les agents hydratants, les vitamines, les agents répulsifs contre les insectes, les parfums, les conservateurs, les tensioactifs, les anti-inflammatoires, les antagonistes de substance P, les charges, les polymères, les propulseurs, les agents alcalinisants ou acidifiants, les colorants ou tout autre ingrédient habituellement utilisé en cosmétique, en particulier pour la fabrication de compositions antisolaires sous forme d'émulsions.

Les corps gras peuvent être constitués par une huile ou une cire ou leurs mélanges. Par huile, on entend un composé liquide à température ambiante. Par cire, on entend un composé solide ou substantiellement solide à température ambiante, et dont le point de fusion est généralement supérieur à 35°C. Ils comprennent également les acides gras, les alcools gras et les esters d'acides gras, linéaires ou cycliques tels que les dérivés d'acide benzoïque, trimellitique et hydroxy-benzoïque.

Comme huiles, on peut citer les huiles minérales (paraffine); végétales (huile d'amande douce, de macadamia, de pépin de cassis, de jojoba) ; synthétiques comme le perhydrosqualène, les alcools, les acides ou les esters gras (comme le benzoate d'alcools en C₁₂-C₁₅ vendu sous la dénomination commerciale « Finsolv TN » par la société Finetex, le palmitate d'octyle, le lanolate d'isopropyle, les triglycérides dont ceux des acides caprique/caprylique), les esters et éthers gras oxyéthylénés ou oxypropylénés; siliconées (cyclométhicone, polydiméthysiloxanes ou PDMS) ou fluorées, les polyalkylènes.

Comme composés cireux, on peut citer la paraffine, la cire de carnauba, la cire d'abeille, l'huile de ricin hydrogénée.

Parmi les solvants organiques, on peut citer les alcools et polyols inférieurs.

Bien entendu, l'homme de l'art veillera à choisir ce ou ces éventuels composés complémentaires et/ou leurs quantités de manière telle que les propriétés avantageuses, en particulier la photostabilité, attachées intrinsèquement aux compositions conformes à l'invention ne soient pas, ou substantiellement pas, altérées par la ou les adjonctions envisagées.

Les compositions de l'invention peuvent être préparées selon les techniques bien connues de l'homme de l'art, en particulier celles destinées à la préparation d'émulsions de type huile-dans-eau ou eau-dans-huile.

Ces compositions peuvent se présenter en particulier sous forme d'émulsion, simple ou complexe (H/E, E/H, H/E/H ou E/H/E) telle qu'une crème, un lait, un gel ou un gel crème, de poudre, de bâtonnet solide et éventuellement être conditionnée en aérosol et se présenter sous forme de mousse ou de spray.

Lorsqu'il s'agit d'une évulsion, la phase aqueuse de celle-ci peut comprendre une dispersion vésiculaire non ionique préparée selon des procédés connus (Bangham, Standish and Watkins. J. Mol. Biol. 13, 238 (1965), FR2315991 et FR2416008).

La composition cosmétique de l'invention peut être utilisée comme composition protectrice de l'épiderme humain ou des cheveux contre les rayons ultraviolets, comme composition antisolaire ou comme produit de maquillage.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de bâtonnet solide, de poudre, de stick, de mousse aérosol ou de spray.

Lorsque la composition cosmétique selon l'invention est utilisée pour la protection des cheveux contre les rayons UV, elle peut se présenter sous forme de shampooing, de lotion, de gel, d'émulsion, de dispersion vésiculaire non ionique et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse (comprenant notamment les filtres hydrophiles) représente généralement de 50 à 95% en poids, de préférence de 70 à 90% en poids, par rapport à l'ensemble de la formulation, la phase huileuse (comprenant notamment les filtres lipophiles) de 5 à 50% en poids, de préférence de 10 à 30% en poids, par rapport à l'ensemble de la formulation, et le ou les (co)émulsionnant(s) de 0,5 à 20% en poids, de préférence de 2 à 10% en poids, par rapport à l'ensemble de la formulation.

Un autre objet de la présente invention réside dans un procédé pour améliorer la stabilité d'au moins un dérivé du dibenzoylméthane vis-à-vis du rayonnement UV consistant à associer au dit dérivé de dibenzoylméthane une quantité efficace d'au moins un dérivé de 2-hydroxybenzophénone aminosubstitué tel que défini ci-dessus. Ce procédé est défini à la revendication 17.

Des exemples concrets illustrant l'invention, vont maintenant être donnés.

| **Exemple 1** | |
|---|---|
| Mélange d'alcool cétylstéarylique et d'alcool cétylstéarylique oxyéthyléné (33 OE) 80/20 (SINNOWAX AO -HENKEL) | 7g |
| | |
| Mélange de mono et distéarate de glycérol (GERASYNT SD-V ISP) | 2g |
| Alcool cétylique | 1.5g |
| | |
| Polydiméthyl siloxane (DOW CORNING 200 FLUID -DOW CORNING) | |
| Benzoate d'alcools en C₁₂-C₁₅ (WITCONCOL TN -WITCO) | 1g 15g |
| | |
| 2-(4-diethylamino-2-hydroxybenzoyl)-benzoate de n-hexyle | 2g |
| | |
| Butyl methoxydibenzoylmethane (PARSOL 1789 - HOFFMAN - LAROCHE) | 1.5g |
| | |
| Glycérine | 10g |
| | |
| Conservateurs | qs |
| | |
| Eau déminéralisée qsp | 100 g |

| **Exemple 2** | |
|---|---|
| Mélange mono /distéarate de glycerol / stéarate de polyéthylène glycol (100 OE) (ARLACEL 165 FL - ICI) | 2g |
| | |
| Alcool stéarylique (LANETTE 18 - HENKEL) | 1g |
| | |
| Acide stéarique d'huile de palme (STEARINE TP - STEARINERIE DUBOIS) | 2.5g |
| | |
| Poly diméthylsiloxane (DOW CORNING 200 FLUID - DOW CORNING) | 0.5g |
| | |
| Benzoate d'alcools en C12/C15 (WITCONOL TN -WITCO) | 15g |
| Triéthanolamine | 0.5g |
| | |
| 2-(4-diethylamino-2-hydroxybenzoyl)-benzoate de n-hexyle | 1.5g |
| | |
| Butyl methoxydibenzoylmethane (PARSOL 1789 - HOFFMAN - LAROCHE) | 1g |
| | |
| Glycérine | 5g |
| | |
| Phosphate d'alcool hexadécylique, sel de potassium (AMPHISOL K - HOFFMAN LAROCHE) | 1g |
| | |
| Acide polyacrylique (SYNTHALEN K -3V) | 0.3g |
| | |
| Hydroxypropyl méthyl cellulose (METHOCEL F4M -DOW CHEMICAL) | 0.1 |
| | |
| Triethanolamine | qs pH 7 |
| | |
| Conservateurs | |
| | |
| Eau démineralisée qsp | 100 g |

## Revendications (Revendications pour l'(les) Etat(s) contractant(s) suivant(s): AT, BE, BG, CH, CY, CZ, DK, EE, ES, FI, FR, GB, GR, IE, IT, LI, LU, MC, NL, PT, SE, SK, TR)

1. Composition cosmétique ou dermatologique, à usage topique, **caractérisée par le fait qu'**elle comprend, dans un support cosmétiquement acceptable, au moins :
a) le 4-(ter.-butyl) 4'-méthoxy dibenzoylméthane et
b) le 2-(4-diéthylamino-2-hydroxybenzoyl)-benzoate de n-hexyle ;
ladite composition ne contenant pas de p-méthylbenzylidène camphre ; le rapport en poids du dérivé de 2-hydroxybenzophénone sur le dérivé de dibenzoylméthane étant supérieur à 1, et le dérivé de dibenzoylméthane étant présent à des teneurs allant de 2 % à 15 % en poids, par rapport au poids total de la composition ; sous réserve que la composition soit différente des formulations suivantes dans lesquelles les quantités sont exprimées en pourcentage en poids par rapport au poids total de la composition :
| **Ingrédients** | **Composition 6** |
|---|---|
| Cetyl Dimethicone Copolyol | 0,5 |
| Polyricinoléate de polyglycérol | 5,0 |
| 2-(4-diéthylamino-2-hydroxybenzoyl)-benzoate de n-hexyle | 5,0 |
| Phénylbenzimidazole, acide sulfonique | 2,5 |
| Octocrylène | 5,0 |
| Diméthicone-diéthylbenzylmalonate | 4,0 |
| Homosalate | 4,0 |
| Butyl méthoxydibenzoylméthane | 3,0 |
| Oxyde de Zinc micronisé | 5,0 |
| Coco-caprylate/caprate | 10,0 |
| Diméthicone | 2,5 |
| Polydécène | 7,0 |
| Polyisobutène | 2,0 |
| Chlorure de sodium | 0,45 |
| Parfum | 0,15 |
| Glycine soja | 2,0 |
| Phénoxyéthanol | 1,0 |
| Eau | qsp 100 |
| **Ingrédients** | **Composition 9** |
|---|---|
| Monostéarate de glycérol autoémulsionnant | 3,00 |
| Polyoxyéthylène(30)cétylstéaryléther | 1,00 |
| Alcool stéarylique | 3,00 |
| Butyl Méthoxydibenzoylméthane | 2,00 |
| Ethylhexyl Triazone | 3,00 |
| 2-(4-diéthylamino-2-hydroxybenzoyl)-benzoate de n-hexyle | 3,50 |
| Acide phénylèn-1,4-bis(monosodium)-2-benzimidazolyl-5,7-disulfonique | 0,5 |
| Oxyde de dioctyle | 3,50 |
| Dicaprylyl Carbonate | 6,00 |
| Diméthicone Polydiméthylsiloxane | 1,00 |
| Glycérine | 5,00 |
| Acétate de tocophéryle | 0,25 |
| Acide dioique | 0,20 |
| Diéthylhexyl-2,6-naphthalate | 2,00 |
| Alpha-Glucosylrutin | 0,20 |
| Paraben | 0,50 |
| Konkaben LMB® | 0,40 |
| Ethanol | 1,50 |
| Parfum | qs |
| Eau | qsp 100 |
| **Ingrédients** | **Composition 12** |
|---|---|
| PEG-30-dipolyhydroxystéarate | 5,00 |
| Butyl méthoxydibenzoylméthane | 2,00 |
| Ethylhexyl Triazone | 3,00 |
| 2-(4-diéthylamino-2-hydroxybenzoyl)-benzoate de n-hexyle | 4,50 |
| Octocrylène | 4,00 |
| Acide phénylèn-1,4-bis(monosodium)-2-benzimidazolyl-5,7-disulfonique | 0,50 |
| Diméthicone-diéthylbenzylmalonate | 5,50 |
| Dioxyde de Titane | 1,50 |
| Oxyde de Zinc | 2,00 |
| Huile de paraffine | 10,0 |
| Esters d'acides caprylique et caprique et de butylène glycol | 2,00 |
| Dicaprylyl Carbonate | 6,00 |
| Diméthicone Polydiméthylsiloxane | 1,00 |
| Beurre de karité | 3,00 |
| Diéthylhexyl-2,6-naphthalate | 6,50 |
| Octoxyglycérine | 1,00 |
| Glycine Soja | 1,50 |
| Chlorure de magnésium | 1,00 |
| Acétate de tocophéryle | 0,25 |
| Acide Dioique | 0,50 |
| Paraben | 0,50 |
| Konkaben LMB® | 0,40 |
| Ethanol | 1,50 |
| Parfum | qs |
| Eau | qsp 100 |
| **Ingrédients** | **Composition 15** |
|---|---|
| Monostérate de glycérine SE | 1,50 |
| PEG-100 stéarate | 2,00 |
| Cetearyl sulfate | 0,75 |
| Alcool stéarylique | 2,00 |
| 2-(4-diéthylamino-2-hydroxybenzoyl)- | 4,50 |
| benzoate de n-hexyle | |
| Ethyhexyl Triazone | 2,00 |
| Drométrizole Trisiloxane | 1,00 |
| Butyl méthoxydibenzoylméthane | 2,00 |
| Acide téréphtalylidène dicamphre sulfonique | 0,50 |
| Oxyde de Zinc HP1® | 2,00 |
| C₁₂-C₁₅ Alkyl Benzoate | 7,00 |
| Dicaprylylcarbonate | 2,00 |
| Glycine Soja | 1,50 |
| Phénoxyéthanol | 0,40 |
| Ethanol | 4,50 |
| Parfum | 0,40 |
| Eau | qsp 100 |
| **Ingrédients** | **Composition 24** |
|---|---|
| Monostérate de glycérine SE | 1,50 |
| PEG-40 stéarate | 2,00 |
| Cetearylsulfate | 0,75 |
| Alcool stéarylique | 2,00 |
| 2-(4-diéthylamino-2-hydroxybenzoyl)-benzoate de n-hexyle | 4,50 |
| Butyl méthoxydibenzoylméthane | 2,00 |
| Ethyhexyl Triazone | 2,00 |
| Drométrizole Trisiloxane | 1,00 |
| Acide téréphtalylidène dicamphre sulfonique | 0,50 |
| Diéthylhexyl-2,6-naphthalate | 5,50 |
| Oxyde de Zinc HP1® | 2,00 |
| C₁₂-C₁₅ Alkyl Benzoate | 7,00 |
| Dicaprylylcarbonate | 2,00 |
| Glycine Soja | 1,50 |
| Phénoxyéthanol | 0,40 |
| Ethanol | 4,50 |
| Parfum | 0,40 |
| Eau | qsp 100 |
| **Ingrédients** | **Composition 37** |
|---|---|
| Glycéryl Stéarate Citrate | 1,00 |
| PEG-100 stéarate | 2,00 |
| Cétylphosphate | 0,75 |
| Alcool stéarylique | 2,00 |
| Dermacryl 79® | 2,00 |
| 2-(4-diéthylamino-2-hydroxybenzoyl)-benzoate de n-hexyle | 4,50 |
| Butyl méthoxydibenzoylméthane | 2,00 |
| Ethyhexyl Triazone | 2,00 |
| Drométrizole Trisiloxane | 1,00 |
| Acide téréphtalylidène dicamphre sulfonique | 0,50 |
| Diéthylhexyl-2,6-naphthalate | 10,00 |
| Oxyde de Zinc HP1® | 2,00 |
| C₁₂-C₁₅ Alkyl Benzoate | 7,00 |
| Dicaprylylcarbonate | 2,00 |
| Glycine Soja | 1,50 |
| Phénoxyéthanol | 0,40 |
| Ethanol | 4,50 |
| Parfum | 0,40 |
| Eau | Qsp 100 |
| **Ingrédients** | **Composition 44** |
|---|---|
| Monostérate de glycérine SE | 0,50 |
| Glycéryl Stéarate Citrate | 2,00 |
| PEG-40 Stéarate | 0,50 |
| 2-(4-diéthylamino-2-hydroxybenzoyl)-benzoate de n-hexyle | 4,00 |
| Butyl méthoxydibenzoylméthane | 2,00 |
| Ethylhexyl triazone | 4,00 |
| Parsol SLX® | 3,50 |
| Mexoryl SX® | 0,25 |
| Bisimidazylate | 1,00 |
| Phénylbenzimidazole, acide sulfonique | 0,50 |
| Dioxyde de Titane MT100 TV® | 1,00 |
| Esters d'acides caprylique et caprique et de butylène glycol | 5,00 |
| Cyclométhicone | 2,00 |
| PVP/Eicosène Copolymer | 0,50 |
| Glycérine | 3,00 |
| Propylène Glycol | 1,50 |
| Gomme de Xanthane | 0,15 |
| Acétate de vitamine E | 0,50 |
| Alpha-glucosylrutine | 0,35 |
| Glycine Soja | 1,00 |
| Pentasodium Ethylèndiamintétraméthylphosphonate (PETP) | 0,25 |
| EDTA Trisodium | 0,10 |
| Méthylparabène | 0,15 |
| Phénoxyéthanol | 1,00 |
| Parfum | 0,20 |
| Eau | qsp 100 |
| **Ingrédients** | **Composition 47** |
|---|---|
| Lauryl Diméthicone Copolyol | 1,00 |
| PEG-30-dipolyhydroxystéarate | 5,00 |
| 2-(4-diéthylamino-2-hydroxybenzoyl)-benzoate de n-hexyle | 5,00 |
| Méthylène Bis-Benzotriazolyl Tétraméthylbutylphénol | 2,00 |
| Butyl méthoxy-dibenzoylméthane | 2,00 |
| Ethylhexyl Triazone | 3,00 |
| Parsol SLX® | 3,50 |
| Octocrylène | 8,00 |
| Bisimidazylate | 0,50 |
| Dioxyde de Titane MT-100 Z | 3,00 |
| Oxyde de Zinc Z-Cote HP1® | 6,00 |
| Huile minérale | 10,00 |
| Esters d'acides caprylique et caprique et de butylène glycol | 2,00 |
| Cyclométhicone | 5,00 |
| Pentasodium Ethylèndiamintétraméthylphosphonate (PETP) | 0,25 |
| Ethylhexyloxyglycérine | 1,00 |
| Glycine Soja | 1,50 |
| Butylène glycol | 10,00 |
| MgCl₂ | 1,00 |
| Vitamine E | 0,25 |
| Ethanol | 4,50 |
| Parfum | 0,20 |
| Eau | qsp 100 |

2. Composition selon la revendication 1, dans laquelle le dérivé de 2-hydroxybenzophénone aminosubstitué est présent dans des proportions allant de 0,1 % à 15 % en poids

3. Composition selon la revendication 2, dans laquelle le dérivé de 2-hydroxybenzophénone aminosubstituéest présent dans des proportions allant de 1 à 10% en poids

4. Composition selon la revendication 3, dans laquelle le dérivé de 2-hydroxybenzophénone aminosubstitué est présent dans des proportions allant de 2 à 8% en poids par rapport au poids total de la composition.

5. Composition selon l'une quelconque des revendications 1 à 4, où le dérivé du dibenzoylméthane est présent à des teneurs allant de 0,5 % à 15 % en poids, par rapport au poids total de la composition.

6. Composition selon la revendication 5, où le dérivé du dibenzoylméthane est présent à des teneurs allant de 1 % à 10% en poids, par rapport au poids total de la composition.

7. Composition selon l'une quelconque des revendications 1 à 6, **caractérisée par le fait qu'**elle contient en plus d'autres filtres organiques actifs dans l'UV-A et/ou l'UV-B.

8. Composition selon la revendication 7, où le ou les filtres UV organiques complémentaires sont choisis parmi les anthranilates ; les dérivés cinnamiques ; les dérivés salicyliques, les dérivés du camphre autres que le p-méthylbenzylidènecamphre ; les dérivés de la benzophénone autres que ceux de formule (I) ; les dérivés de β,β'-diphénylacrylate, les dérivés de benzotriazole ; les dérivés de benzalmalonate ; les dérivés de benzimidazole ; les imadazolines ; les dérivés bis-benzoazolyle ; les dérivés de l'acide p-aminobenzoïque (PABA) ; les dérivés de méthylène bis-(hydroxyphényl benzotriazole) ; les polymères filtres et silicones filtres ; les dimères dérivés d'α-alkylstyrène ; les 4,4-diarylbutadiènes.

9. Composition selon la revendication 8, **caractérisée par le fait que** le ou les filtres UV organiques sont choisis parmi les composés suivants :
- Ethylhexyl Salicylate,
- Ethylhexyl Methoxycinnamate
- Octocrylene,
- Phenylbenzimidazole Sulfonic Acid,
- Terephthalylidene Dicamphor Sulfonic,.
- Benzophenone-3,
- Benzophenone-4,
- Benzophenone-5,
- 4-Methylbenzylidene camphor,
- Disodium Phenyl Dibenzimidazole Tetra-sulfonate
- Anisotriazine,
- Ethylhexyl triazone,
- Diethylhexyl Butamido Triazone,
- la 2,4,6-tris-(4'-amino benzalmalonate de diisobutyle)-s-triazine
- Methylene bis-Benzotriazolyl Tetramethylbutylphenol,
- Drometrizole Trisiloxane,
et leurs mélanges.

10. Composition selon l'une quelconque des revendications 1 à 9, **caractérisée par le fait qu'**elle comprend en outre, des pigments ou des nanopigments d'oxydes métalliques, enrobés ou non.

11. Composition selon la revendication 10, **caractérisée par le fait que** lesdits pigments ou nanopigments sont choisis parmi les oxydes de titane, de zinc, de fer, de zirconium, de cérium et leurs mélanges, enrobés ou non.

12. Composition selon l'une quelconque des revendications 1 à 11, **caractérisée par le fait qu'**elle comprend en outre au moins un agent de bronzage et/ou de brunissage artificiel de la peau.

13. Composition selon l'une quelconque des revendications 1 à 12, **caractérisée par le fait qu'**elle comprend en outre au moins un adjuvant choisi parmi les corps gras, les solvants organiques, les épaississants ioniques ou non ioniques, les adoucissants, les antioxydants, les agents anti radicaux libres, les opacifiants, les stabilisants, les émollients, les silicones, les α-hydroxyacides, les agents anti-mousse, les agents hydratants, les vitamines, les agents répulsifs contre les insectes, les parfums, les conservateurs, les tensioactifs, les anti-inflammatoires, les antagonistes de substance P, les charges, les polymères, les propulseurs, les agents alcalinisants ou acidifiants, les colorants.

14. Composition selon l'une quelconque des revendications 1 à 13, **caractérisée par le fait qu'**il s'agit d'une composition protectrice de l'épiderme humain ou d'une composition antisolaire et qu'elle se présente sous forme d'une dispersion vésiculaire non ionique, d'une émulsion, en particulier d'une émulsion de type huile-dans-eau, d'une crème, d'un lait, d'un gel, d'un gel crème, d'une suspension, d'une dispersion, d'une poudre, d'un bâtonnet solide, d'une mousse ou d'un spray.

15. Composition selon l'une quelconque des revendications 1 à 13, **caractérisée par le fait qu'**il s'agit d'une composition de maquillage des cils, des sourcils ou de la peau et qu'elle se présente sous forme solide ou pâteuse, anhydre ou aqueuse, d'une émulsion, d'une suspension ou d'une dispersion.

16. Composition selon l'une quelconque des revendications 1 à 13, **caractérisée par le fait qu'**il s'agit d'une composition destinée à la protection des cheveux contre les rayons ultraviolets et qu'elle se présente sous la forme d'un shampooing, d'une lotion, d'un gel, d'une émulsion, d'une dispersion vésiculaire non ionique.

17. Procédé pour améliorer la stabilité vis-à-vis du rayonnement UV d'au moins un dérivé du dibenzoylméthane dans une composition cosmétique ou dermatolologique ne contenant pas de p-méthylbenzylidènecamphre consistant à associer audit dérivé de dibenzoylméthane une quantité efficace d'au moins un dérivé de 2-hydroxybenzophénone aminosubstitué tel que défini dans l'une quelconque des revendications précédentes ; le rapport en poids du dérivé de 2-hydroxybenzophénone sur le dérivé de dibenzoylméthane étant supérieur à 1.

## Revendications (Revendications pour l'(les) Etat(s) contractant(s) suivant(s): DE)

1. Composition cosmétique ou dermatologique, à usage topique, **caractérisée par le fait qu'**elle comprend, dans un support cosmétiquement acceptable, au moins :
a) le 4-(ter.-butyl) 4'-méthoxy dibenzoylméthane et
b) le 2-(4-diéthylamino-2-hydroxybenzoyl)-benzoate de n-hexyle
; ladite composition ne contenant pas de p-méthylbenzylidène camphre ; le rapport en poids du dérivé de 2-hydroxybenzophénone sur le dérivé de dibenzoylméthane étant supérieur à 1, et le dérivé de dibenzoylméthane étant présent à des teneurs allant de 2 % à 15 % en poids, par rapport au poids total de la composition ; sous réserve que la composition soit différente des formulations suivantes dans lesquelles les quantités sont exprimées en pourcentage en poids par rapport au poids total de la composition :
| **Ingrédients** | **Composition 6** |
|---|---|
| Cetyl Dimethicone Copolyol | 0,5 |
| Polyricinoléate de polyglycérol | 5,0 |
| 2-(4-diéthylamino-2-hydroxybenzoyl)-benzoate de n-hexyle | 5,0 |
| Phénylbenzimidazole, acide sulfonique | 2,5 |
| Octocrylène | 5,0 |
| Diméthicone-diéthylbenzylmalonate | 4,0 |
| Homosalate | 4,0 |
| Butyl méthoxydibenzoylméthane | 3,0 |
| Oxyde de Zinc micronisé | 5,0 |
| Coco-caprylate/caprate | 10,0 |
| Diméthicone | 2,5 |
| Polydécène | 7,0 |
| Polyisobutène | 2,0 |
| Chlorure de sodium | 0,45 |
| Parfum | 0,15 |
| Glycine soja | 2,0 |
| Phénoxyéthanol | 1,0 |
| Eau | qsp 100 |
| **Ingrédients** | **Composition 9** |
|---|---|
| Monostéarate de glycérol autoémulsionnant | 3,00 |
| Polyoxyéthylène(30)cétylstéaryléther | 1,00 |
| Alcool stéarylique | 3,00 |
| Butyl Méthoxydibenzoylméthane | 2,00 |
| Ethylhexyl Triazone | 3,00 |
| 2-(4-diéthylamino-2-hydroxybenzoyl)-benzoate de n-hexyle | 3,50 |
| Acide phénylèn-1,4-bis(monosodium)-2-benzimidazolyl-5,7-disulfonique | 0,5 |
| Oxyde de dioctyle | 3,50 |
| Dicaprylyl Carbonate | 6,00 |
| Diméthicone Polydiméthylsiloxane | 1,00 |
| Glycérine | 5,00 |
| Acétate de tocophéryle | 0,25 |
| Acide dioique | 0,20 |
| Diéthylhexyl-2,6-naphthalate | 2,00 |
| Alpha-Glucosylrutin | 0,20 |
| Paraben | 0,50 |
| Konkaben LMB® | 0,40 |
| Ethanol | 1,50 |
| Parfum | qs |
| Eau | qsp 100 |
| **Ingrédients** | **Composition 12** |
|---|---|
| PEG-30-dipolyhydroxystéarate | 5,00 |
| Butyl méthoxydibenzoylméthane | 2,00 |
| Ethylhexyl Triazone | 3,00 |
| 2-(4-diéthylamino-2-hydroxybenzoyl)-benzoate de n-hexyle | 4,50 |
| Octocrylène | 4,00 |
| Acide phénylèn-1,4-bis(monosodium)-2-benzimidazolyl-5,7-disulfonique | 0,50 |
| Diméthicone-diéthylbenzylmalonate | 5,50 |
| Dioxyde de Titane | 1,50 |
| Oxyde de Zinc | 2,00 |
| Huile de paraffine | 10,0 |
| Esters d'acides caprylique et caprique et de butylène glycol | 2,00 |
| Dicaprylyl Carbonate | 6,00 |
| Diméthicone Polydiméthylsiloxane | 1,00 |
| Beurre de karité | 3,00 |
| Diéthylhexyl-2,6-naphthalate | 6,50 |
| Octoxyglycérine | 1,00 |
| Glycine Soja | 1,50 |
| Chlorure de maqnésium | 1,00 |
| Acétate de tocophéryle | 0,25 |
| Acide Dioique | 0,50 |
| Paraben | 0,50 |
| Konkaben LMB® | 0,40 |
| Ethanol | 1,50 |
| Parfum | qs |
| Eau | qsp 100 |
| **Ingrédients** | **Composition 15** |
|---|---|
| Monostérate de glycérine SE | 1,50 |
| PEG-100 stéarate | 2,00 |
| Cetearyl sulfate | 0,75 |
| Alcool stéarylique | 2,00 |
| 2-(4-diéthylamino-2-hydroxybenzoyl)-benzoate de n-hexyle | 4,50 |
| Ethyhexyl Triazone | 2,00 |
| Drométrizole Trisiloxane | 1,00 |
| Butyl méthoxydibenzoylméthane | 2,00 |
| Acide téréphtalylidène dicamphre sulfonique | 0,50 |
| Oxyde de Zinc HP1® | 2,00 |
| C₁₂-C₁₅ Alkyl Benzoate | 7,00 |
| Dicaprylylcarbonate | 2,00 |
| Glycine Soja | 1,50 |
| Phénoxyéthanol | 0,40 |
| Ethanol | 4,50 |
| Parfum | 0,40 |
| Eau | qsp 100 |
| **Ingrédients** | **Composition 24** |
|---|---|
| Monostérate de glycérine SE | 1,50 |
| PEG-40 stéarate | 2,00 |
| Cetearylsulfate | 0,75 |
| Alcool stéarylique | 2,00 |
| 2-(4-diéthylamino-2-hydroxybenzoyl)-benzoate de n-hexyle | 4,50 |
| Butyl méthoxydibenzoylméthane | 2,00 |
| Ethyhexyl Triazone | 2,00 |
| Drométrizole Trisiloxane | 1,00 |
| Acide téréphtalylidène dicamphre sulfonique | 0,50 |
| Diéthylhexyl-2,6-naphthalate | 5,50 |
| Oxyde de Zinc HP1® | 2,00 |
| C₁₂-C₁₅ Alkyl Benzoate | 7,00 |
| Dicaprylylcarbonate | 2,00 |
| Glycine Soja | 1,50 |
| Phénoxyéthanol | 0,40 |
| Ethanol | 4,50 |
| Parfum | 0,40 |
| Eau | qsp 100 |
| **Ingrédients** | **Composition 37** |
|---|---|
| Glycéryl Stéarate Citrate | 1,00 |
| PEG-100 stéarate | 2,00 |
| Cétylphosphate | 0,75 |
| Alcool stéarylique | 2,00 |
| Dermacryl 79® | 2,00 |
| 2-(4-diéthylamino-2-hydroxybenzoyl)-benzoate de n-hexyle | 4,50 |
| Butyl méthoxydibenzoylméthane | 2,00 |
| Ethyhexyl Triazone | 2,00 |
| Drométrizole Trisiloxane | 1,00 |
| Acide téréphtalylidène dicamphre sulfonique | 0,50 |
| Diéthylhexyl-2,6-naphthalate | 10,00 |
| Oxyde de Zinc HP1® | 2,00 |
| C₁₂-C₁₅ Alkyl Benzaote | 7,00 |
| Dicaprylylcarbonate | 2,00 |
| Glycine Soja | 1,50 |
| Phénoxyéthanol | 0,40 |
| Ethanol | 4,50 |
| Parfum | 0,40 |
| Eau | Qsp 100 |
| **Ingrédients** | **Composition 44** |
|---|---|
| Monostérate de glycérine SE | 0,50 |
| Glycéryl Stéarate Citrate | 2,00 |
| PEG-40 Stéarate | 0,50 |
| 2-(4-diéthylamino-2-hydroxybenzoyl)-benzoate de n-hexyle | 4,00 |
| Butyl méthoxydibenzoylméthane | 2,00 |
| Ethylhexyl triazone | 4,00 |
| Parsol SLX® | 3,50 |
| Mexoryl SX® | 0,25 |
| Bisimidazylate | 1,00 |
| Phénylbenzimidazole, acide sulfonique | 0,50 |
| Dioxyde de Titane MT100 TV® | 1,00 |
| Esters d'acides caprylique et caprique et de butylène glycol | 5,00 |
| Cyclométhicone | 2,00 |
| PVP/Eicosène Copolymer | 0,50 |
| Glycérine | 3,00 |
| Propylène Glycol | 1,50 |
| Gomme de Xanthane | 0,15 |
| Acétate de vitamine E | 0,50 |
| Alpha-qlucosylrutine | 0,35 |
| Glycine Soja | 1,00 |
| Pentasodium Ethylèndiamintétraméthylphosphonate (PETP) | 0,25 |
| EDTA Trisodium | 0,10 |
| Méthylparabène | 0,15 |
| Phénoxyéthanol | 1,00 |
| Parfum | 0,20 |
| Eau | qsp 100 |
| **Ingrédients** | **Composition 47** |
|---|---|
| Lauryl Diméthicone Copolyol | 1,00 |
| PEG-30-dipolyhydroxystéarate | 5,00 |
| 2-(4-diéthylamino-2-hydroxybenzoyl)-benzoate de n-hexyle | 5,00 |
| Méthylène Bis-Benzotriazolyl Tétraméthylbutylphénol | 2,00 |
| Butyl méthoxy-dibenzoylméthane | 2,00 |
| Ethyhexyl Triazone | 3 ,00 |
| Parsol SLX® | 3,50 |
| Octocrylène | 8,00 |
| Bisimidazylate | 0,50 |
| Dioxyde de Titane MT-100 Z | 3,00 |
| Oxyde de Zinc Z-Cote HP1® | 6,00 |
| Huile minérale | 10,00 |
| Esters d'acides caprylique et caprique et de butylène glycol | 2,00 |
| Cyclométhicone | 5,00 |
| Pentasodium Ethylèndiamintétraméthylphosphonate (PETP) | 0,25 |
| Ethylhexyloxyglycérine | 1,00 |
| Glycine Soja | 1,50 |
| Butylène glycol | 10,00 |
| MgCl₂ | 1,00 |
| Vitamine E | 0,25 |
| Ethanol | 4,50 |
| Parfum | 0,20 |
| Eau | qsp 100 |
| **Ingrédients** | **Composition 51** |
|---|---|
| PEG-30-dipolyhydroxystéarate | 5,00 |
| Diméthicone/Vinyl Diméthicone Crosspolymer | 5,00 |
| 2-(4-diéthylamino-2-hydroxybenzoyl)-benzoate de n-hexyle | 3,00 |
| Méthylène Bis-Benzotriazolyl Tétraméthylbutylphénol | 2,00 |
| Butyl méthoxy-dibenzoylméthane | 2,00 |
| Ethyhexyl Triazone | 3,00 |
| PARSOL SLX® | 3,50 |
| Octocrylène | 8,00 |
| Bisimidazylate | 0,50 |
| Dioxyde de Titane MT-100 Z® | 3,00 |
| Oxyde de Zinc Z-Cote HP1® | 6,00 |
| Huile minérale | 10,00 |
| Esters d'acides caprylique et caprique et de butylène glycol | 2,00 |
| Diméthicone | 12,50 |
| 2,6-Diéthylhexylnaphthalate | 5,50 |
| Ethylhexyloxyglycérine | 1,00 |
| Butylène glycol | 10,00 |
| Glycine Soja | 1,50 |
| MgCl₂ | 1,00 |
| Vitamine E | 0,25 |
| Ethanol | 4,50 |
| Parfum | 0,20 |
| Eau | qsp 100 |
| **Ingrédients** | **Composition 53** |
|---|---|
| Monostérate de glycérine SE | 0,50 |
| Glycéryl Stéarate Citrate | 2,00 |
| PEG-40 stéarate | 0,50 |
| Vinyldiméthicone/Méthicone Silesquioxane Crosspolymer (KSP100 Shin Etsu) | 10,00 |
| 2-(4-diéthylamino-2-hydroxybenzoyl)-benzoate de n-hexyle | 4,00 |
| Butyl Méthoxydibenzoylméthane | 2,00 |
| Ethylhexyl Triazone | 4,00 |
| PARSOL SLX® | 3,50 |
| Mexoryl SX® | 0,25 |
| Acide phényl Dibenzimidazol Tétrasulfonique | 1,00 |
| Phénylbenzimidazol, acide sulfonique | 0,50 |
| Dioxyde de titane MT 100 TV® | 1,00 |
| Esters d'acides caprylique et caprique et de butylène glycol | 5,00 |
| Cyclométhicone | 2,00 |
| PVP/Hexadécène Copolymer | 0,50 |
| Glycérine | 3,00 |
| Gomme de Xanthane | 0,15 |
| Acétate de vitamine E | 0,50 |
| Alpha-qlucosylrutine | 0,35 |
| 2,6-Diéthylhexylnaphthalate | 4,00 |
| Trisodium EDTA | 0,10 |
| Méthylparabène | 0,15 |
| Phénoxyéthanol | 1,00 |
| Parfum | 0,20 |
| Eau | qsp 100 |
| **Ingrédients** | **Composition 56** |
|---|---|
| PEG-30-dipolyhydroxystéarate | 5,00 |
| Vinyldiméthicone/Méthicone Silesquioxane Crosspolymer modifié par des groupes fluoroalkyles (KSP200, Shin Etsu) | 2,75 |
| 2-(4-diéthylamino-2-hydroxybenzoyl)-benzoate de n-hexyle | 3,00 |
| Méthylène Bis-Benzotriazolyl Tétraméthylbutylphénol | 2,00 |
| Butyl méthoxydibenzoylméthane | 2,00 |
| Ethyhexyl Triazone | 3,00 |
| PARSOL SLX® | 3,50 |
| Acide phényl Dibenzimidazol Tétrasulfonique | 0,50 |
| Dioxyde de Titane MT-100 Z® | 3,00 |
| Oxyde de Zinc Z-Cote HP1® | 6,00 |
| Huile minérale | 10,00 |
| Esters d'acides caprylique et caprique et de butylène glycol | 2,00 |
| Diméthicone | 12,50 |
| 2,6-Diéthylhexylnaphthalate | 5,50 |
| Ethylhexyloxyglycérine Butylène glycol | 1,00 |
| | 10,00 |
| Glycine Soja | 1,50 |
| MgCl₂ | 1,00 |
| Vitamine E | 0,25 |
| Ethanol | 4,50 |
| Parfum | 0,20 |
| Eau | qsp 100 |

2. Composition selon la revendication 1, dans laquelle le dérivé de 2-hydroxybenzophénone aminosubstitué est présent dans des proportions allant de 0,1 % à 15 % en poids.

3. Composition selon la revendication 2, dans laquelle le dérivé de 2-hydroxybenzophénone aminosubstitué est présent dans des proportions allant de 1 à 10% en poids.

4. Composition selon la revendication 3, dans laquelle le dérivé de 2-hydroxybenzophénone aminosubstitué est présent dans des proportions allant de 2 à 8% en poids par rapport au poids total de la composition.

5. Composition selon l'une quelconque des revendications 1 à 4, où le dérivé du dibenzoylméthane est présent à des teneurs allant de 0,5 % à 15 % en poids, par rapport au poids total de la composition.

6. Composition selon la revendication 5, où le dérivé du dibenzoylméthane est présent à des teneurs allant de 1 % à 10% en poids, par rapport au poids total de la composition.

7. Composition selon l'une quelconque des revendications 1 à 6, **caractérisée par le fait qu'**elle contient en plus d'autres filtres organiques actifs dans l'UV-A et/ou l'UV-B.

8. Composition selon la revendication 7, où le ou les filtres UV organiques complémentaires sont choisis parmi les anthranilates ; les dérivés cinnamiques ; les dérivés salicyliques, les dérivés du camphre autres que le p-méthylbenzylidènecamphre ; les dérivés de la benzophénone autres que ceux de formule (I) ; les dérivés de β,β'-diphénylacrylate, les dérivés de benzotriazole ; les dérivés de benzalmalonate ; les dérivés de benzimidazole ; les imadazolines ; les dérivés bis-benzoazolyle ; les dérivés de l'acide p-aminobenzoïque (PABA) ; les dérivés de méthylène bis-(hydroxyphényl benzotriazole) ; les polymères filtres et silicones filtres ; les dimères dérivés d'α-alkylstyrène ; les 4,4-diarylbutadiènes.

9. Composition selon la revendication 8, **caractérisée par le fait que** le ou les filtres UV organiques sont choisis parmi les composés suivants :
- Ethylhexyl Salicylate,
- Ethylhexyl Methoxycinnamate
- Octocrylene,
- Phenylbenzimidazole Sulfonic Acid,
- Terephthalylidene Dicamphor Sulfonic,.
- Benzophenone-3,
- Benzophenone-4,
- Benzophenone-5,
- 4-Methylbenzylidene camphor,
- Disodium Phenyl Dibenzimidazole Tetra-sulfonate
- Anisotriazine,
- Ethylhexyl triazone,
- Diethylhexyl Butamido Triazone,
- la 2,4,6-tris-(4'-amino benzalmalonate de diisobutyle)-s- triazine
- Methylene bis-Benzotriazolyl Tetramethylbutylphenol,
- Drometrizole Trisiloxane,
et leurs mélanges.

10. Composition selon l'une quelconque des revendications 1 à 9, **caractérisée par le fait qu'**elle comprend en outre, des pigments ou des nanopigments d'oxydes métalliques, enrobés ou non.

11. Composition selon la revendication 10, **caractérisée par le fait que** lesdits pigments ou nanopigments sont choisis parmi les oxydes de titane, de zinc, de fer, de zirconium, de cérium et leurs mélanges, enrobés ou non.

12. Composition selon l'une quelconque des revendications 1 à 11, **caractérisée par le fait qu'**elle comprend en outre au moins un agent de bronzage et/ou de brunissage artificiel de la peau.

13. Composition selon l'une quelconque des revendications 1 à 12, **caractérisée par le fait qu'**elle comprend en outre au moins un adjuvant choisi parmi les corps gras, les solvants organiques, les épaississants ioniques ou non ioniques, les adoucissants, les antioxydants, les agents anti radicaux libres, les opacifiants, les stabilisants, les émollients, les silicones, les α-hydroxyacides, les agents anti-mousse, les agents hydratants, les vitamines, les agents répulsifs contre les insectes, les parfums, les conservateurs, les tensioactifs, les anti-inflammatoires, les antagonistes de substance P, les charges, les polymères, les propulseurs, les agents alcalinisants ou acidifiants, les colorants.

14. Composition selon l'une quelconque des revendications 1 à 13, **caractérisée par le fait qu'**il s'agit d'une composition protectrice de l'épiderme humain ou d'une composition antisolaire et qu'elle se présente sous forme d'une dispersion vésiculaire non ionique, d'une émulsion, en particulier d'une émulsion de type huile-dans-eau, d'une crème, d'un lait, d'un gel, d'un gel crème, d'une suspension, d'une dispersion, d'une poudre, d'un bâtonnet solide, d'une mousse ou d'un spray.

15. Composition selon l'une quelconque des revendications 1 à 13, **caractérisée par le fait qu'**il s'agit d'une composition de maquillage des cils, des sourcils ou de la peau et qu'elle se présente sous forme solide ou pâteuse, anhydre ou aqueuse, d'une émulsion, d'une suspension ou d'une dispersion.

16. Composition selon l'une quelconque des revendications 1 à 13, **caractérisée par le fait qu'**il s'agit d'une composition destinée à la protection des cheveux contre les rayons ultraviolets et qu'elle se présente sous la forme d'un shampooing, d'une lotion, d'un gel, d'une émulsion, d'une dispersion vésiculaire non ionique.

17. Procédé pour améliorer la stabilité vis-à-vis du rayonnement UV d'au moins un dérivé du dibenzoylméthane dans une composition cosmétique ou dermatolologique ne contenant pas de p-méthylbenzylidènecamphre consistant à associer audit dérivé de dibenzoylméthane une quantité efficace d'au moins un dérivé de 2-hydroxybenzophénone aminosubstitué tel que défini dans l'une quelconque des revendications précédentes : le rapport en poids du dérivé de 2-hydroxybenzophénone sur le dérivé de dibenzoylméthane étant supérieur à 1.

## Patentansprüche (Patentansprüche für folgende(n) Vertragsstaat(en): AT, BE, BG, CH, CY, CZ, DK, EE, ES, FI, FR, GB, GR, IE, IT, LI, LU, MC, NL, PT, SE, SK, TR)

1. Kosmetische oder dermatologische Zusammensetzung für die topische Verwendung, **dadurch gekennzeichnet, dass** sie in einem kosmetisch akzeptablen Träger mindestens Folgendes umfasst:
(a) 4-(tert-Butyl)-4'-methoxydibenzoylmethan und
(b) n-Hexyl-2-(4-diethylamino-2-hydroxybenzoyl)-benzoat;
wobei die Zusammensetzung keinen p-Methylbenzylidenkampfer enthält; wobei das Verhältnis, bezogen auf Gewicht, des 2-Hydroxybenzophenon-Derivats zum Dibenzoylmethan-Derivat größer als 1 ist und das Dibenzoylmethan-Derivat in Konzentrationen von 2 bis 15 Gew.-%, bezogen auf das Gesamtgewicht der Zusammensetzung, vorliegt; mit der Maßgabe, dass die Zusammensetzung verschieden von den nachstehenden Formulierungen ist, in denen die Mengen als Gewichtsprozent, bezogen auf das Gesamtgewicht der Zusammensetzung, ausgedrückt sind:
| **Inhaltsstoffe** | **Zusammensetzung 6** |
|---|---|
| Cetyl-Dimethicon-Copolyol | 0,5 |
| Polyglycerin-Polyricinoleat | 5,0 |
| n-Hexyl-2-(4-diethylamino-2-hydroxybenzoyl)-benzoat | 5,0 |
| Phenylbenzimidazol, Sulfonsäure | 2,5 |
| Octocrylen | 5,0 |
| Dimethicon-Diethylbenzylmalonat | 4,0 |
| Homosalat | 4,0 |
| Butylmethoxydibenzoylmethan | 3,0 |
| mikronisiertes Zinkoxid | 5,0 |
| Coco-Caprylat/-Caprat | 10,0 |
| Dimethicon | 2,5 |
| Polydecen | 7,0 |
| Polyisobuten | 2,0 |
| Natriumchlorid | 0,45 |
| Parfum | 0,15 |
| Glycine soja | 2,0 |
| Phenoxyethanol | 1,0 |
| Wasser | qs 100 |
| **Inhaltsstoffe** | **Zusammensetzung 9** |
|---|---|
| selbstemulgierendes Glycerinmonostearat | 3,00 |
| Polyoxyethylen(30)cetylstearylether | 1,00 |
| Stearylalkohol | 3,00 |
| Butylmethoxydibenzoylmethan | 2,00 |
| Ethylhexyltriazon | 3,00 |
| n-Hexyl-2-(4-diethylamino-2-hydroxybenzoyl)-benzoat | 3,50 |
| Phenylen-1,4-bis(mononatrium)-2-benzimidazolyl-5,7-disulfonsäure | 0,5 |
| Dioctyloxid | 3,50 |
| Dicaprylylcarbonat | 6,00 |
| Dimethicon-Polydimethylsiloxan | 1,00 |
| Glycerin | 5,00 |
| Tocopherylacetat | 0,25 |
| Dionsäure | 0,20 |
| Diethylhexyl-2,6-naphthalat | 2,00 |
| alpha-Glucosylrutin | 0,20 |
| Paraben | 0,50 |
| Konkaben LMB^{®} | 0,40 |
| Ethanol | 1,50 |
| Parfum | qs |
| Wasser | qs 100 |
| **Inhaltsstoffe** | **Zusammensetzung 12** |
|---|---|
| PEG-30-dipolyhydroxystearat | 5,00 |
| Butylmethoxydibenzoylmethan | 2,00 |
| Ethylhexyltriazon | 3,00 |
| n-Hexyl-2-(4-diethylamino-2-hydroxybenzoyl)-benzoat | 4,50 |
| Octocrylen | 4,00 |
| Phenylen-1,4-bis(mononatrium)-2-benzimidazolyl-5,7-disulfonsäure | 0,50 |
| Dimethicon-Diethylbenzylmalonat | 5,50 |
| Titandioxid | 1,50 |
| Zinkoxid | 2,00 |
| Paraffinöl | 10,0 |
| Ester von Capryl- und Caprinsäure und Butylenglykol | 2,00 |
| Dicaprylylcarbonat | 6,00 |
| Dimethicon-Polydimethylsiloxan | 1,00 |
| Karitébutter | 3,00 |
| Diethylhexyl-2,6-naphthalat | 6,50 |
| Octoxyglycerin | 1,00 |
| Glycine soja | 1,50 |
| Magnesiumchlorid | 1,00 |
| Tocopherylacetat | 0,25 |
| Dionsäure | 0,50 |
| Paraben | 0,50 |
| Konkaben LMB^{®} | 0,40 |
| Ethanol | 1,50 |
| Parfum | qs |
| Wasser | qs 100 |
| **Inhaltsstoffe** | **Zusammensetzung 15** |
|---|---|
| Glycerinmonostearat SE | 1,50 |
| PEG-100-stearat | 2,00 |
| Cetearylsulfat | 0,75 |
| Stearylalkohol | 2,00 |
| n-Hexyl-2-(4-diethylamino-2-hydroxybenzoyl)-benzoat | 4,50 |
| Ethylhexyltriazon | 2,00 |
| Drometrizoltrisiloxan | 1,00 |
| Butylmethoxydibenzoylmethan | 2,00 |
| Terephthalylidendikampfersulfonsäure | 0,50 |
| Zinkoxid HP1^{®} | 2,00 |
| C₁₂-C₁₅-Alkylbenzoat | 7,00 |
| Dicaprylylcarbonat | 2,00 |
| Glycine soja | 1,50 |
| Phenoxyethanol | 0,40 |
| Ethanol | 4,50 |
| Parfum | 0,40 |
| Wasser | qs 100 |
| **Inhaltsstoffe** | **Zusammensetzung 24** |
|---|---|
| Glycerinmonostearat SE | 1,50 |
| PEG-40-stearat | 2,00 |
| Cetearylsulfat | 0,75 |
| Stearylalkohol | 2,00 |
| n-Hexyl-2-(4-diethylamino-2-hydroxybenzoyl)-benzoat | 4,50 |
| Butylmethoxydibenzoylmethan | 2,00 |
| Ethylhexyltriazon | 2,00 |
| Drometrizoltrisiloxan | 1,00 |
| Terephthalylidendikampfersulfonsäure | 0,50 |
| Diethylhexyl-2,6-naphthalat | 5,50 |
| Zinkoxid HP1^{®} | 2,00 |
| C₁₂-C₁₅-Alkylbenzoat | 7,00 |
| Dicaprylylcarbonat | 2,00 |
| Glycine soja | 1,50 |
| Phenoxyethanol | 0,40 |
| Ethanol | 4,50 |
| Parfum | 0,40 |
| Wasser | qs 100 |
| **Inhaltsstoffe** | **Zusammensetzung 37** |
|---|---|
| Glycerylstearatcitrat | 1,00 |
| PEG-100-stearat | 2,00 |
| Cetylphosphat | 0,75 |
| Stearylalkohol | 2,00 |
| Dermacryl 79^{®} | 2,00 |
| n-Hexyl-2-(4-diethylamino-2-hydroxybenzoyl)-benzoat | 4,50 |
| Butylmethoxydibenzoylmethan | 2,00 |
| Ethylhexyltriazon | 2,00 |
| Drometrizoltrisiloxan | 1,00 |
| Terephthalylidendikampfersulfonsäure | 0,50 |
| Diethyhexyl-2,5-naphthalat | 10,00 |
| Zinkoxid HP1^{®} | 2,00 |
| C₁₂-C₁₅-Alkylbenzoat | 7,00 |
| Dicaprylylcarbonat | 2,00 |
| Glycine soja | 1,50 |
| Phenoxyethanol | 0,40 |
| Ethanol | 4,50 |
| Parfum | 0,40 |
| Wasser | qs 100 |
| **Inhaltsstoffe** | **Zusammensetzung 44** |
|---|---|
| Glycerinmonostearat SE | 0,50 |
| Glycerylstearatcitrat | 2,00 |
| PEG-40-stearat | 0,50 |
| n-Hexyl-2-(4-diethylamino-2-hydroxybenzoyl)-benzoat | 4,00 |
| Butylmethoxydibenzoylmethan | 2,00 |
| Ethylhexyltriazon | 4,00 |
| Parsol SLX^{®} | 3,50 |
| Mexoryl SX^{®} | 0,25 |
| Bisimidazylat | 1,00 |
| Phenylbenzimidazol, Sulfonsäure | 0,50 |
| Titandioxid MT100 TV^{®} | 1,00 |
| Ester von Capryl- und Capronsäure und Butylenglykol | 5,00 |
| Cyclomethicon | 2,00 |
| PVP/Eicosen-Copolymer | 0,50 |
| Glycerin | 3,00 |
| Propylenglykol | 1,50 |
| Xanthangummi | 0,15 |
| Vitamin E-acetat | 0,50 |
| alpha-Glucosylrutin | 0,35 |
| Glycine soja | 1,00 |
| Pentanatriumethylendiamintetramethylphosphonat (PETP) | 0,25 |
| Trinatrium-EDTA | 0,10 |
| Methylparaben | 0,15 |
| Phenoxyethanol | 1,00 |
| Parfum | 0,20 |
| Wasser | qs 100 |
| **Inhaltsstoffe** | **Zusammensetzung** 47 |
|---|---|
| Lauryl-Dimethicon-Copolymer | 1,00 |
| PEG-30-dipolyhydroxystearat | 5,00 |
| n-Hexyl-2-(4-diethylamino-2-hydroxybenzoyl)-benzoat | 5,00 |
| Methylenbisbenzotriazolyltetramethylbutylphenol | 2,00 |
| Butylmethoxydibenzoylmethan | 2,00 |
| Ethylhexyltriazon | 3,00 |
| Parsol SLX^{®} | 3,50 |
| Octocrylen | 8,00 |
| Bisimidazylat | 0,50 |
| Titandioxid MT-100 Z | 3,00 |
| Zinkoxid Z-Cote HP1^{®} | 6,00 |
| Mineralöl | 10,00 |
| Ester von Capryl- und Capronsäure und Butylenglykol | 2,00 |
| Cyclomethicon | 5,00 |
| Pentanatriumethylendiamintetramethylphosphonat (PETP) | 0,25 |
| Ethylhexyloxyglycerin | 1,00 |
| Glycine soja | 1,50 |
| Butylenglykol | 10,00 |
| MgCl₂ | 1,00 |
| Vitamin E | 0,25 |
| Ethanol | 4,50 |
| Parfum | 0,20 |
| Wasser | qs 100 |

2. Zusammensetzung nach Anspruch 1, wobei das aminosubstituierte 2-Hydroxybenzophenon-Derivat in Anteilen von 0,1 bis 15 Gew.-% vorliegt.

3. Zusammensetzung nach Anspruch 2, wobei das aminosubstituierte 2-Hydroxybenzophenon-Derivat in Anteilen von 1 bis 10 Gew.-% vorliegt.

4. Zusammensetzung nach Anspruch 3, wobei das aminosubstituierte 2-Hydroxybenzophenon-Derivat in Anteilen von 2 bis 8 Gew.-%-, bezogen auf das Gesamtgewicht der Zusammensetzung, vorliegt.

5. Zusammensetzung nach einem der Ansprüche 1 bis 4, wobei das Derivat von Dibenzoylmethan in Konzentrationen von 0,5 bis 15 Gew.-%, bezogen auf das Gesamtgewicht der Zusammensetzung, vorliegt.

6. Zusammensetzung nach Anspruch 5, wobei das Derivat von Dibenzoylmethan in Konzentrationen von 1 bis 10 Gew.-%, bezogen auf das Gesamtgewicht der Zusammensetzung, vorliegt.

7. Zusammensetzung nach einem der Ansprüche 1 bis 6, **dadurch gekennzeichnet, dass** sie außerdem andere organische Filter enthält, die im UV-A- und/oder UV-B-Bereich aktiv sind.

8. Zusammensetzung nach Anspruch 7, wobei der oder die zusätzliche(n) organische(n) UV-Filter aus Anthranilaten; Zimtsäure-Derivaten; Salicylsäure-Derivaten; anderen Kampfer-Derivaten als p-Methylbenzylidenkampfer; anderen Benzophenon-Derivaten als denjenigen der Formel (I); β,β-Diphenylacrylat-Derivaten; Benzotriazol-Derivaten; Benzalmalonat-Derivaten; Benzimidazol-Derivaten; Imidazolinen; Bisbenzoazolyl-Derivaten; p-Aminobenzoesäure-(PABA)-Derivaten; Methylenbis(hydroxyphenylbenzotriazol)-Derivaten; Filter-Polymeren und Filter-Silikonen; von α-Alkylstyrol herrührenden Dimeren; 4,4-Diarylbutadienen ausgewählt ist/sind.

9. Zusammensetzung nach Anspruch 8, **dadurch gekennzeichnet, dass** der oder die organische(n) UV-Filter aus den folgenden Verbindungen ausgewählt ist/sind:
- Ethylhexylsalicylat,
- Ethylhexylmethoxycinnamat,
- Octocrylen,
- Phenylbenzimidazolsulfonsäure,
- Terephthalylidendikampfersulfonsäure,
- Benzophenon-3,
- Benzophenon-4,
- Benzophenon-5,
- 4-Methylbenzylidenkampfer,
- Dinatriumphenyldibenzimidazoltetrasulfonat,
- Anisotriazin,
- Ethylhexyltriazon,
- Diethylhexylbutamidotriazon,
- 2,4,6-Tris-(diisobutyl-4'-aminobenzalmalonat)-s-triazin,
- Methylenbisbenzotriazolyltetramethylbutylphenol,
- Drometrizoltrisiloxan
und deren Gemischen.

10. Zusammensetzung nach einem der Ansprüche 1 bis 9, **dadurch gekennzeichnet, dass** sie außerdem überzogene oder nicht überzogene Metalloxidpigmente oder nanopigmente umfasst.

11. Zusammensetzung nach Anspruch 10, **dadurch gekennzeichnet, dass** die Pigmente oder Nanopigmente aus überzogenen oder nicht überzogenen Titan-, Zink-, Eisen-, Zirkon- oder Ceroxiden und deren Gemischen ausgewählt sind.

12. Zusammensetzung nach einem der Ansprüche 1 bis 11, **dadurch gekennzeichnet, dass** sie außerdem mindestens ein Mittel zur künstlichen Bräunung und/oder Braunfärbung der Haut umfasst.

13. Zusammensetzung nach einem der Ansprüche 1 bis 12, **dadurch gekennzeichnet, dass** sie außerdem mindestens einen Hilfsstoff umfasst, der aus Fettsubstanzen, organischen Lösungsmitteln, ionischen oder nicht-ionischen Verdickungsmitteln, Enthärtungsmitteln, Antioxidantien, Mitteln gegen freie Radikale, Trübungsmitteln, Stabilisatoren, Weichmachern, Silikonen, α-Hydroxysäuren, Antischaummitteln, Hydratisierungsmitteln, Vitaminen, Insektenabwehrmitteln, Parfums, Konservierungsmitteln, Tensiden, entzündungshemmenden Mitteln, Antagonisten von Substanz P, Füllstoffen, Polymeren, Treibmitteln, Alkalinisierungsmitteln oder Ansäuerungsmitteln, Färbemitteln ausgewählt ist.

14. Zusammensetzung nach einem der Ansprüche 1 bis 13, **dadurch gekennzeichnet, dass** es sich um eine Schutzzusammensetzung für die menschliche Epidermis oder eine Sonnenschutzzusammensetzung handelt und dass sie in Form einer nicht-ionischen Vesikeldispersion, einer Emulsion, insbesondere einer Emulsion des Öl-in-Wasser-Typs, einer Creme, einer Milch, eines Gels, eines Cremegels, einer Suspension, einer Dispersion, eines Pulvers, eines festen Stiftes, eines Schaums oder eines Sprays vorliegt.

15. Zusammensetzung nach einem der Ansprüche 1 bis 13, **dadurch gekennzeichnet, dass** es sich um eine Zusammensetzung zum Schminken der Wimpern, der Augenbrauen oder der Haut handelt und dass sie in fester oder pastöser, wasserfreier oder wässriger Form, in Form einer Emulsion, einer Suspension oder einer Dispersion vorliegt.

16. Zusammensetzung nach einem der Ansprüche 1 bis 13, **dadurch gekennzeichnet, dass** es sich um eine für den Schutz der Haare gegen ultraviolette Strahlen bestimmte Zusammensetzung handelt und dass sie in Form eines Shampoos, einer Lotion, eines Gels, einer Emulsion, einer nicht-ionischen Vesikeldispersion vorliegt.

17. Verfahren zur Verbesserung der Beständigkeit mindestens eines Derivats von Dibenzoylmethan gegenüber UV-Strahlung in einer kosmetischen oder dermatologischen Zusammensetzung, die keinen p-Methylbenzylidenkampfer enthält, bestehend aus dem Zugeben zu dem Dibenzoylmethan-Derivat einer wirksamen Menge von mindestens einem aminosubstituierten 2-Hydroxybenzophenon-Derivat nach einem der vorhergehenden Ansprüche: dabei ist das Gewichtsverhältnis des 2-Hydroxybenzophenon-Derivats zum Dibenzoylmethan-Derivat größer als 1.

## Patentansprüche (Patentansprüche für folgende(n) Vertragsstaat(en): DE)

1. Kosmetische oder dermatologische Zusammensetzung für die topische Verwendung, **dadurch gekennzeichnet, dass** sie in einem kosmetisch akzeptablen Träger mindestens Folgendes umfasst:
(a) 4-(tert-Butyl)-4'-methoxydibenzoylmethan und
(b) n-Hexyl-2-(4-diethylamino-2-hydroxybenzoyl)-benzoat;
wobei die Zusammensetzung keinen p-Methylbenzylidenkampfer enthält; wobei das Verhältnis, bezogen auf Gewicht, des 2-Hydroxybenzophenon-Derivats zum Dibenzoylmethan-Derivat größer als 1 ist und das Dibenzoylmethan-Derivat in Konzentrationen von 2 bis 15 Gew.-%, bezogen auf das Gesamtgewicht der Zusammensetzung, vorliegt; mit der Maßgabe, dass die Zusammensetzung verschieden von den nachstehenden Formulierungen ist, in denen die Mengen als Gewichtsprozent, bezogen auf das Gesamtgewicht der Zusammensetzung, ausgedrückt sind:
| **Inhaltsstoffe** | **Zusammensetzung 6** |
|---|---|
| Cetyl-Dimethicon-Copolyol | 0,5 |
| Polyglycerin-Polyricinoleat | 5,0 |
| n-Hexyl-2-(4-diethylamino-2-hydroxybenzoyl)-benzoat | 5,0 |
| Phenylbenzimidazol, Sulfonsäure | 2,5 |
| Octocrylen | 5,0 |
| Dimethicon-Diethylbenzylmalonat | 4,0 |
| Homosalat | 4,0 |
| Butylmethoxydibenzoylmethan | 3,0 |
| mikronisiertes Zinkoxid | 5,0 |
| Coco-Caprylat/-Caprat | 10,0 |
| Dimethicon | 2,5 |
| Polydecen | 7,0 |
| Polyisobuten | 2,0 |
| Natriumchlorid | 0,45 |
| Parfum | 0,15 |
| Glycine soja | 2,0 |
| Phenoxyethanol | 1,0 |
| Wasser | qs 100 |
| **Inhaltsstoffe** | **Zusammensetzung 9** |
|---|---|
| selbstemulgierendes Glycerinmonostearat | 3,00 |
| Polyoxyethylen(30)cetylstearylether | 1,00 |
| Stearylalkohol | 3,00 |
| Butylmethoxydibenzoylmethan | 2,00 |
| Ethylhexyltriazon | 3,00 |
| n-Hexyl-2-(4-diethylamino-2-hydroxybenzoyl)-benzoat | 3,50 |
| Phenylen-1,4-bis(mononatrium)-2-benzimidazolyl-5,7-disulfonsäure | 0,5 |
| Dioctyloxid | 3,50 |
| Dicaprylylcarbonat | 6,00 |
| Dimethicon-Polydimethylsiloxan | 1,00 |
| Glycerin | 5,00 |
| Tocopherylacetat | 0,25 |
| Dionsäure | 0,20 |
| Diethylhexyl-2,6-naphthalat | 2,00 |
| alpha-Glucosylrutin | 0,20 |
| Paraben | 0,50 |
| Konkaben LMB^{®} | 0,40 |
| Ethanol | 1,50 |
| Parfum | qs |
| Wasser | qs 100 |
| **Inhaltsstoffe** | **Zusammensetzung 12** |
|---|---|
| PEG-30-dipolyhydroxystearat | 5,00 |
| Butylmethoxydibenzoylmethan | 2,00 |
| Ethylhexyltriazon | 3,00 |
| n-Hexyl-2-(4-diethylamino-2-hydroxybenzoyl)-benzoat | 4,50 |
| Octocrylen | 4,00 |
| Phenylen-1,4-bis(mononatrium)-2-benzimidazolyl-5,7-disulfonsäure | 0,50 |
| Dimethicon-Diethylbenzylmalonat | 5,50 |
| Titandioxid | 1,50 |
| Zinkoxid | 2,00 |
| Paraffinöl | 10,0 |
| Ester von Capryl- und Caprinsäure und Butylenglykol | 2,00 |
| Dicaprylylcarbonat | 6,00 |
| Dimethicon-Polydsmethylsiloxan | 1,00 |
| Karitebutter | 3,00 |
| Diethylhexyl-2,6-naphthalat | 6,50 |
| Octoxyglycerin | 1,00 |
| Glycine soja | 1,50 |
| Magnesiumchlorid | 1,00 |
| Tocopherylacetat | 0,25 |
| Dionsäure | 0,50 |
| Paraben | 0,50 |
| Konkaben LMB^{®} | 0,40 |
| Ethanol | 1,50 |
| Parfum | qs |
| Wasser | qs 100 |
| **Inhaltsstoffe** | **Zusammensetzung 15** |
|---|---|
| Glycerinmonostearat SE | 1,50 |
| PEG-100-stearat | 2,00 |
| Cetearylsulfat | 0,75 |
| Stearylalkohol | 2,00 |
| n-Hexyl-2-(4-diethylamino-2-hydroxybenzoyl)-benzoat | 4,50 |
| Ethylhexyltriazon | 2,00 |
| Drometrizoltrisiloxan | 1,00 |
| Butylmethoxydibenzoylmethan | 2,00 |
| Terephthalylidendikampfersulfonsäure | 0,50 |
| Zinkoxid HP1^{®} | 2,00 |
| C₁₂-C₁₅-Alkylbenzoat | 7,00 |
| Dicaprylylcarbonat | 2,00 |
| Glycine soja | 1,50 |
| Phenoxyethanol | 0,40 |
| Ethanol | 4,50 |
| Parfum | 0,40 |
| Wasser | qs 100 |
| **Inhaltsstoffe** | **Zusammensetzung 24** |
|---|---|
| Glycerinmonostearat SE | 1,50 |
| PEG-40-stearat | 2,00 |
| Cetearylsulfat | 0,75 |
| Stearylalkohol | 2,00 |
| n-Hexyl-2-(4-diethylamino-2-hydroxybenzoyl)-benzoat | 4,50 |
| Butylmethoxydibenzoylmethan | 2,00 |
| Ethylhexyltriazon | 2,00 |
| Drometrizoltrisiloxan | 1,00 |
| Terephthalylidendikampfersulfonsäure | 0,50 |
| Diethylhexyl-2,6-naphthalat | 5,50 |
| Zinkoxid HP1^{®} | 2,00 |
| C₁₂-C₁₅-Alkylbenzoat | 7,00 |
| Dicaprylylcarbonat | 2,00 |
| Glycine soja | 1,50 |
| Phenoxyethanol | 0,40 |
| Ethanol | 4,50 |
| Parfum | 0,40 |
| Wasser | qs 100 |
| **Inhaltsstoffe** | **Zusammensetzung 37** |
|---|---|
| Glycerylstearatcitrat | 1,00 |
| PEG-100-stearat | 2,00 |
| Cetylphosphat | 0,75 |
| Stearylalkohol | 2,00 |
| Dermacryl 79^{®} | 2,00 |
| n-Hexyl-2-(4-diethylamino-2-hydroxybenzoyl)-benzoat | 4,50 |
| Butylmethoxydibenzoylmethan | 2,00 |
| Ethylhexyltriazon | 2,00 |
| Drometrizoltrisiloxan | 1,00 |
| Terephthalylidendikampfersulfonsäure | 0,50 |
| Diethylhexyl-2,6-naphthalat | 10,00 |
| Zinkoxid HP1^{®} | 2,00 |
| C₁₂-C₁₅-Alkylbenzoat | 7,00 |
| Dicaprylylcarbonat | 2,00 |
| Glycine soja | 1,50 |
| Phenoxyethanol | 0,40 |
| Ethanol | 4,50 |
| Parfum | 0,40 |
| Wasser | qs 100 |
| **Inhaltsstoffe** | **Zusammensetzung 44** |
|---|---|
| Glycerinmonostearat SE | 0,50 |
| Glycerylstearatcitrat | 2,00 |
| PEG-40-stearat | 0,50 |
| n-Hexyl-2-(4-diethylamino-2-hydroxybenzoyl)-benzoat | 4,00 |
| Butylmethoxydibenzoylmethan | 2,00 |
| Ethylhexyltriazon | 4,00 |
| Parsol SLX^{®} | 3,50 |
| Mexoryl SX^{®} | 0,25 |
| Bisimidazylat | 1,00 |
| Phenylbenzimidazol, Sulfonsäure | 0,50 |
| Titandioxid MT100 TV^{®} | 1,00 |
| Ester von Capryl- und Capronsäure und Butylenglykol | 5,00 |
| Cyclomethicon | 2,00 |
| PVP/Eicosen-Copolymer | 0,50 |
| Glycerin | 3,00 |
| Propylenglykol | 1,50 |
| Xanthangummi | 0,15 |
| Vitamin E-acetat | 0,50 |
| alpha-Glucosylrutin | 0,35 |
| Glycine soja | 1,00 |
| Pentanatriumethylendiamintetramethylphosphonat (PETP) | 0,25 |
| Trinatrium-EDTA | 0,10 |
| Methylparaben | 0,15 |
| Phenoxyethanol | 1,00 |
| Parfum | 0,20 |
| Wasser | qs 100 |
| **Inhaltsstoffe** | **Zusammensetzung 47** |
|---|---|
| Lauryl-Dimethicon-Copolymer | 1,00 |
| PEG-30-dipolyhydroxystearat | 5,00 |
| n-Hexyl-2-(4-diethylamino-2-hydroxybenzoyl)-benzoat | 5,00 |
| Methylenbisbenzotriazolyltetramethylbutylphenol | 2,00 |
| Butylmethoxydibenzoylmethan | 2,00 |
| Ethylhexyltriazon | 3,00 |
| Parsol SLX^{®} | 3,50 |
| Octocrylen | 8,00 |
| Bisimidazylat | 0,50 |
| Titandioxid MT-100 Z | 3,00 |
| Zinkoxid Z-Cote HP1^{®} | 6,00 |
| Mineralöl | 10,00 |
| Ester von Capryl- und Capronsäure und Butylenglykol | 2,00 |
| Cyclomethicon | 5,00 |
| Pentanatriumethylendiamintetramethylphosphonat (PETP) | 0,25 |
| Ethylhexyloxyglycerin | 1,00 |
| Glycine soja | 1,50 |
| Butylenglykol | 10,00 |
| MgCl₂ | 1,00 |
| Vitamin E | 0,25 |
| Ethanol | 4,50 |
| Parfum | 0,20 |
| Wasser | qs 100 |
| **Inhaltsstoffe** | **Zusammensetzung 51** |
|---|---|
| PEG-30-dipolyhydroxystearat | 5,00 |
| Dimethicon/Vinyldimethicon-Kreuzpolymer | 5,00 |
| n-Hexyl-2-(4-diethylamino-2-hydroxybenzoyl)-benzoat | 3,00 |
| Methylenbisbenzotriazolyltetramethylbutylphenol | 2,00 |
| Butylmethoxydibenzoylmethan | 2,00 |
| Ethylhexyltriazon | 3,00 |
| PARSOL SLX^{®} | 3,50 |
| Octocrylen | 8,00 |
| Bisimidazylat | 0,50 |
| Titandioxid MT-100 Z^{®} | 3,00 |
| Zinkoxid Z-Cote HP1^{®} | 6,00 |
| Mineralöl | 10,00 |
| Ester von Capryl- und Capronsäure und Butylenglykol | 2,00 |
| Dimethicon | 12,50 |
| 2,6-Diethylhexylnaphthalat | 5,50 |
| Ethylhexyloxyglycerin | 1,00 |
| Butylenglykol | 10,00 |
| Glycine soja | 1,50 |
| MgCl₂ | 1,00 |
| Vitamin E | 0,25 |
| Ethanol | 4,50 |
| Parfum | 0,20 |
| Wasser | qs 100 |
| **Inhaltsstoffe** | **Zusammensetzung 53** |
|---|---|
| Glycerinmonostearat SE | 0,50 |
| Glycerylstearatcitrat | 2,00 |
| PEG-40-stearat | 0,50 |
| Vinyldimethicon/Methiconsilesquioxan-Kreuzpolymer (KSP100 Shin Etsu) | 10,00 |
| n-Hexyl-2-(4-diethylamino-2-hydroxybenzoyl)-benzoat | 4,00 |
| Butylmethoxydibenzoylmethan | 2,00 |
| Ethylhexyltriazon | 4,00 |
| PARSOL SLX^{®} | 3,50 |
| Mexoryl SX^{®} | 0,25 |
| Phenyldibenzimidazoltetrasulfonsäure | 1,00 |
| Phenylbenzimidazol, Sulfonsäure | 0,50 |
| Titandioxid MT 100 TV^{®} | 1,00 |
| Ester von Capryl- und Capronsäure und Butylenglykol | 5,00 |
| Cyclomethicon | 2,00 |
| PVP/Hexadecen-Copolymer | 0,50 |
| Glycerin | 3,00 |
| Xanthangummi | 0,15 |
| Vitamin E-acetat | 0,50 |
| alpha-Glycosylrutin | 0,35 |
| 2,6-Diethylhexylnaphthalat | 4,00 |
| Trinatrium-EDTA | 0,10 |
| Methylparaben | 0,15 |
| Phenoxyethanol | 1,00 |
| Parfum | 0,20 |
| Wasser | qs 100 |
| **Inhaltsstoffe** | **Zusammensetzung 56** |
|---|---|
| PEG-30-dipolyhydroxystearat | 5,00 |
| Vinyldimethicon/Methiconsilesquioxan-Kreuzpolymer, mit Fluoralkylgruppen modifiziert (KSP200 Shin Etsu) | 2,75 |
| n-Hexyl-2-(4-diethylamino-2-hydroxybenzoyl)-benzoat | 3,00 |
| Methylenbisbenzotriazolyltetramethylbutylphenol | 2,00 |
| Butylmethoxydibenzoylmethan | 2,00 |
| Ethylhexyltriazon | 3,00 |
| PARSOL SLX^{®} | 3,50 |
| Phenyldibenzimidazoltetrasulfonsäure | 0,50 |
| Titandioxid MT-100 Z^{®} | 3,00 |
| Zinkoxid Z-Cote HP1^{®} | 6,00 |
| Mineralöl | 10,00 |
| Ester von Capryl- und Capronsäure und Butylenglykol | 2,00 |
| Dimethicon | 12,50 |
| 2,6-Diethylhexylnaphthalat | 5,50 |
| Ethylhexyloxyglycerin | 1,00 |
| Butylenglykol | 10,00 |
| Glycine soja | 1,50 |
| MgCl₂ | 1,00 |
| Vitamin E | 0,25 |
| Ethanol | 4,50 |
| Parfum | 0,20 |
| Wasser | qs 100 |

2. Zusammensetzung nach Anspruch 1, wobei das aminosubstituierte 2-Hydroxybenzophenon-Derivat in Anteilen von 0,1 bis 15 Gew.-% vorliegt.

3. Zusammensetzung nach Anspruch 2 wobei das aminosubstituierte 2-Hydroxybenzophenon-Derivat in Anteilen von 1 bis 10 Gew.-% vorliegt

4. Zusammensetzung nach Anspruch 3, wobei das aminosubstituierte 2-Hydroxybenzophenon-Derivat in Anteilen von 2 bis 8 Gew.-%, bezogen auf das Gesamtgewicht der Zusammensetzung, vorliegt.

5. Zusammensetzung nach einem der Ansprüche 1 bis 4, wobei das Derivat von Dibenzoylmethan in Konzentrationen von 0,5 bis 15 Gew.-%, bezogen auf das Gesamtgewicht der Zusammensetzung, vorliegt.

6. Zusammensetzung nach Anspruch 5, wobei das Derivat von Dibenzoylmethan in Konzentrationen von 1 bis 10 Gew.-%, bezogen auf das Gesamtgewicht der Zusammensetzung, vorliegt.

7. Zusammensetzung nach einem der Ansprüche 1 bis 6, **dadurch gekennzeichnet, dass** sie außerdem andere organische Filter enthält, die im UV-A- und/oder UV-B-Bereich aktiv sind.

8. Zusammensetzung nach Anspruch 7, wobei der oder die zusätzliche(n) organische(n) UV-Filter aus Anthranilaten; Zimtsäure-Derivaten; Salicylsäure-Derivaten; anderen Kampfer-Derivaten als p-Methylbenzylidenkampfer; anderen Benzophenon-Derivaten als denjenigen der Formel (I); β,β-Diphenylacrylat-Derivaten; Benzotriazol-Derivaten; Benzalmalonat-Derivaten; Benzimidazol-Derivaten; Imidazolinen; Bisbenzoazolyl-Derivaten; p-Aminobenzoesäure-(PABA)-Derivaten; Methylenbis(hydroxyphenylbenzotriazol)-Derivaten; Filter-Polymeren und Filter-Silikonen; von α-Alkylstyrol herrührenden Dimeren; 4,4-Diarylbutadienen ausgewählt ist/sind.

9. Zusammensetzung nach Anspruch 8, **dadurch gekennzeichnet, dass** der oder die organische(n) UV-Filter aus den folgenden Verbindungen ausgewählt ist/sind:
- Ethylhexylsalicylat,
- Ethylhexylmethoxycinnamat,
- Octocrylen,
- Phenylbenzimidazolsulfonsäure,
- Terephthalylidendikampfersulfonsäure,
- Benzophenon-3,
- Benzophenon-4,
- Benzophenon-5,
- 4-Methylbenzylidenkampfer,
- Dinatriumphenyldibenzimidazoltetrasulfonat,
- Anisotriazin,
- Ethylhexyltriazon,
- Diethylhexylbutamidotriazon,
- 2,4,6-Tris-(diisobutyl-4'-aminobenzalmalonat)-s-triazin,
- Methylenbisbenzotriazolyltetramethylbutylphenol,
- Drometrizoltrisiloxan
und deren Gemischen.

10. Zusammensetzung nach einem der Ansprüche 1 bis 9, **dadurch gekennzeichnet, dass** sie außerdem überzogene oder nicht überzogene Metalloxidpigmente oder - nanopigmente umfasst.

11. Zusammensetzung nach Anspruch 10, **dadurch gekennzeichnet, dass** die Pigmente oder Nanopigmente aus überzogenen oder nicht überzogenen Titan-, Zink-, Eisen-, Zirkon- oder Ceroxiden und deren Gemischen ausgewählt sind.

12. Zusammensetzung nach einem der Ansprüche 1 bis 11, **dadurch gekennzeichnet, dass** sie außerdem mindestens ein Mittel zur künstlichen Bräunung und/oder Braunfärbung der Haut umfasst.

13. Zusammensetzung nach einem der Ansprüche 1 bis 12, **dadurch gekennzeichnet, dass** sie außerdem mindestens einen Hilfsstoff umfasst, der aus Fettsubstanzen, organischen Lösungsmitteln, ionischen oder nichtionischen Verdickungsmitteln, Enthärtungsmitteln, Antioxidantien, Mitteln gegen freie Radikale, Trübungsmitteln, Stabilisatoren, Weichmachern, Silikonen, α-Hydroxysäuren, Antischaummitteln, Hydratisierungsmitteln, Vitaminen, Insektenabwehrmitteln, Parfums, Konservierungsmitteln, Tensiden, entzündungshemmenden Mitteln, Antagonisten von Substanz P, Füllstoffen, Polymeren, Treibmitteln, Alkalinisierungsmitteln oder Ansäuerungsmitteln, Färbemitteln ausgewählt ist.

14. Zusammensetzung nach einem der Ansprüche 1 bis 13, **dadurch gekennzeichnet, dass** es sich um eine Schutzzusammensetzung für die menschliche Epidermis oder eine Sonnenschutzzusammensetzung handelt und dass sie in Form einer nicht-ionischen Vesikeldispersion, einer Emulsion, insbesondere einer Emulsion des Öl-in-Wasser-Typs, einer Creme, einer Milch, eines Gels, eines Cremegels, einer Suspension, einer Dispersion, eines Pulvers, eines festen Stiftes, eines Schaums oder eines Sprays vorliegt.

15. Zusammensetzung nach einem der Ansprüche 1 bis 13, **dadurch gekennzeichnet, dass** es sich um eine Zusammensetzung zum Schminken der Wimpern, der Augenbrauen oder der Haut handelt und dass sie in fester oder pastöser, wasserfreier oder wässriger Form, in Form einer Emulsion, einer Suspension oder einer Dispersion vorliegt.

16. Zusammensetzung nach einem der Ansprüche 1 bis 13, **dadurch gekennzeichnet, dass** es sich um eine für den Schutz der Haare gegen ultraviolette Strahlen bestimmte Zusammensetzung handelt und dass sie in Form eines Shampoos, einer Lotion, eines Gels, einer Emulsion, einer nicht-ionischen Vesikeldispersion vorliegt.

17. Verfahren zur Verbesserung der Beständigkeit mindestens eines Derivats von Dibenzoylmethan gegenüber UV-Strahlung in einer kosmetischen oder dermatologischen Zusammensetzung, die keinen p-Metrylbenzylidenkampfer enthält, bestehend aus dem Zugeben zu dem Dibenzoylmethan-Derivat einer wirksamen Menge von mindestens einem aminosubstituierten 2-Hydroxybenzophenon-Derivat nach einem der vorhergehenden Ansprüche: dabei ist das Gewichtsverhältnis des 2-Hydroxybenzophenon-Derivats zum Dibenzoylmethan-Derivat größer als 1.

## Claims (Claims for the following Contracting State(s): AT, BE, BG, CH, CY, CZ, DK, EE, ES, FI, FR, GB, GR, IE, IT, LI, LU, MC, NL, PT, SE, SK, TR)

1. Cosmetic or dermatological composition, for topical use, **characterized in that** it comprises, in a cosmetically acceptable vehicle, at least:
a) 4-(tert-butyl)-4'-methoxydibenzoylmethane and
b) n-hexyl 2-(4-diethylamino-2-hydroxybenzoylbenzoate;
the said composition not comprising p-methylbenzylidene camphor; the ratio by weight of the 2-hydroxybenzophenone derivative to the dibenzoylmethane derivative being greater than 1 and the dibenzoylmethane derivative being present at contents ranging from 2% to 15% by weight, with respect to the total weight of the composition; with the proviso that the composition is different from the following formulations in which the amounts are expressed as percentages by weight, with respect to the total weight of the composition:
| **Ingredients** | **Composition 6** |
|---|---|
| Cetyl dimethicone copolyol | 0.5 |
| Polyglycerol polyricinoleate | 5.0 |
| n-Hexyl 2-(4-diethylamino-2-hydroxybenzoyl)benzoate | 5.0 |
| Phenylbenzimidazole sulfonic acid | 2.5 |
| Octocrylene | 5.0 |
| Dimethicone-diethyl benzalmalonate | 4.0 |
| Homosalate | 4.0 |
| Butyl methoxydibenzoylmethane | 3.0 |
| Micronized zinc oxide | 5.0 |
| Coco-caprylate/caprate | 10.0 |
| Dimethicone | 2.5 |
| Polydecene | 7.0 |
| Polyisobutene | 2.0 |
| Sodium chloride | 0.45 |
| Fragrance | 0.15 |
| Glycine soja | 2.0 |
| Phenoxyethanol | 1.0 |
| Water | q.s. for 100 |
| **Ingredients** | **Composition 9** |
|---|---|
| Self-emulsifying glycerol monostearate | 3.00 |
| Polyoxyethylene (30) cetyl stearyl ether | 1.00 |
| Stearyl alcohol | 3.00 |
| Butyl methoxydibenzoylmethane | 2.00 |
| Ethylhexyl triazone | 3.00 |
| n-Hexyl 2-(4-diethylamino-2-hydroxybenzoyl)benzoate | 3.50 |
| Phenylene-1,4-bis(2-benzimidazolyl-5,7-disulfonic acid, monosodium salt) | 0.5 |
| Dioctyl oxide | 3.50 |
| Dicaprylyl carbonate | 6.00 |
| Dimethicone polydimethylsiloxane | 1.00 |
| Glycerin | 5.00 |
| Tocopheryl acetate | 0.25 |
| Dioic acid | 0.20 |
| Diethylhexyl 2,6-naphthalate | 2.00 |
| alpha-Glucosylrutin | 0.20 |
| Paraben | 0.50 |
| Konkaben LMB® | 0.40 |
| Ethanol | 1.50 |
| Fragrance | q.s. |
| Water | q.s. for 100 |
| **Ingredients** | **Composition 12** |
|---|---|
| PEG-30 dipolyhydroxystearate | 5.00 |
| Butyl methoxydibenzoylmethane | 2.00 |
| Ethylhexyl triazone | 3.00 |
| n-Hexyl 2-(4-diethylamino-2-hydroxybenzoyl)benzoate | 4.50 |
| Octocrylene | 4.00 |
| Phenylene-1,4-bis(2-benzimidazolyl-5,7-disulfonic acid, monosodium salt) | 0.50 |
| Dimethicone diethyl benzalmalonate | 5.50 |
| Titanium dioxide | 1.50 |
| Zinc oxide | 2.00 |
| Liquid paraffin | 10.0 |
| Esters of caprylic and capric acids and of butylene glycol | 2.00 |
| Dicaprylyl carbonate | 6.00 |
| Dimethicone polydimethylsiloxane | 1.00 |
| Shea butter | 3.00 |
| Diethylhexyl 2,6-naphthalate | 6.50 |
| Octoxyglycerin | 1.00 |
| Glycine soja | 1.50 |
| Magnesium chloride | 1.00 |
| Tocopheryl acetate | 0.25 |
| Dioic acid | 0.50 |
| Paraben | 0.50 |
| Konkaben LMB® | 0.40 |
| Ethanol | 1.50 |
| Fragrance | q.s. |
| Water | q.s. for 100 |
| **Ingredients** | **Composition 15** |
|---|---|
| Glyceryl monostearate SE | 1.50 |
| PEG-100 stearate | 2.00 |
| Cetearyl sulfate | 0.75 |
| Stearyl alcohol | 2.00 |
| n-Hexyl 2-(4-diethylamino-2-hydroxybenzoyl)benzoate | 4.50 |
| Ethylhexyl triazone | 2.00 |
| Drometrizole trisiloxane | 1.00 |
| Butyl methoxydibenzoylmethane | 2.00 |
| Terephthalylidene dicamphor sulfonic acid | 0.50 |
| Zinc oxide HP1® | 2.00 |
| C₁₂-C₁₅ Alkyl benzoate | 7.00 |
| Dicaprylyl carbonate | 2.00 |
| Glycine soja | 1.50 |
| Phenoxyethanol | 0.40 |
| Ethanol | 4.50 |
| Fragrance | 0.40 |
| Water | q.s. for 100 |
| **Ingredients** | **Composition 24** |
|---|---|
| Glyceryl monostearate SE | 1.50 |
| PEG-40 stearate | 2.00 |
| Cetearyl sulfate | 0.75 |
| Stearyl alcohol | 2.00 |
| n-Hexyl 2-(4-diethylamino-2-hydroxybenzoyl)benzoate | 4.50 |
| Butyl methoxydibenzoylmethane | 2.00 |
| Ethylhexyl triazone | 2.00 |
| Drometrizole trisiloxane | 1.00 |
| Terephthalylidene dicamphor sulfonic acid | 0.50 |
| Diethylhexyl 2,6-naphthalate | 5.50 |
| Zinc oxide HP1® | 2.00 |
| C₁₂-C₁₅ Alkyl benzoate | 7.00 |
| Dicaprylyl carbonate | 2.00 |
| Glycine soja | 1.50 |
| Phenoxyethanol | 0.40 |
| Ethanol | 4.50 |
| Fragrance | 0.40 |
| Water | q.s. for 100 |
| **Ingredients** | **Composition 37** |
|---|---|
| Glyceryl stearate citrate | 1.00 |
| PEG-100 stearate | 2.00 |
| Cetyl phosphate | 0.75 |
| Stearyl alcohol | 2.00 |
| Dermacryl 79® | 2.00 |
| n-Hexyl 2-(4-diethylamino-2-hydroxybenzoyl)benzoate | 4.50 |
| Butyl methoxydibenzoylmethane | 2.00 |
| Ethylhexyl triazone | 2.00 |
| Drometrizole trisiloxane | 1.00 |
| Terephthalylidene dicamphor sulfonic acid | 0.50 |
| Diethylhexyl 2,6-naphthalate | 10.00 |
| Zinc oxide HP1® | 2.00 |
| C₁₂-C₁₅ Alkyl benzoate | 7.00 |
| Dicaprylyl carbonate | 2.00 |
| Glycine soja | 1.50 |
| Phenoxyethanol | 0.40 |
| Ethanol | 4.50 |
| Fragrance | 0.40 |
| Water | q.s. for 100 |
| **Ingredients** | **Composition 44** |
|---|---|
| Glyceryl monostearate SE | 0.50 |
| Glyceryl stearate citrate | 2.00 |
| PEG-40 stearate | 0.50 |
| n-Hexyl 2-(4-diethylamino-2-hydroxybenzoyl)benzoate | 4.00 |
| Butyl methoxydibenzoylmethane | 2.00 |
| Ethylhexyl triazone | 4.00 |
| Parsol SLX® | 3.50 |
| Mexoryl SX® | 0.25 |
| Bisimidazylate | 1.00 |
| Phenylbenzimidazole sulfonic acid | 0.50 |
| Titanium dioxide MT100 TV® | 1.00 |
| Esters of caprylic and capric acids and of butylene glycol | 5.00 |
| Cyclomethicone | 2.00 |
| PVP/eicosene copolymer | 0.50 |
| Glycerin | 3.00 |
| Propylene glycol | 1.50 |
| Xanthan gum | 0.15 |
| Vitamin E acetate | 0.50 |
| alpha-Glucosylrutin | 0.35 |
| Glycine soja | 1.00 |
| Pentasodium ethylenediaminetetramethylenephosphonate (PETP) | 0.25 |
| Trisodium EDTA | 0.10 |
| Methylparaben | 0.15 |
| Phenoxyethanol | 1.00 |
| Fragrance | 0.20 |
| Water | q.s. for 100 |
| **Ingredients** | **Composition 47** |
|---|---|
| Lauryl dimethicone copolyol | 1.00 |
| PEG-30 dipolyhydroxystearate | 5.00 |
| n-Hexyl 2-(4-diethylamino-2-hydroxybenzoyl)benzoate | 5.00 |
| Methylene bis-benzotriazolyl tetramethylbutylphenol | 2.00 |
| Butyl methoxydibenzoylmethane | 2.00 |
| Ethylhexyl triazone | 3.00 |
| Parsol SLX® | 3.50 |
| Octocrylene | 8.00 |
| Bisimidazylate | 0.50 |
| Titanium dioxide MT-100 Z | 3.00 |
| Zinc oxide Z-cote HP1® | 6.00 |
| Mineral oil | 10.00 |
| Esters of caprylic and capric acids and of butylene glycol | 2.00 |
| Cyclomethicone | 5.00 |
| Pentasodium ethylenediaminetetramethylenephosphonate (PETP) | 0.25 |
| Ethylhexylglycerin | 1.00 |
| Glycine soja | □.50 |
| Butylene glycol | 10.00 |
| MgCl₂ | 1.00 |
| Vitamin E | 0.25 |
| Ethanol | 4.50 |
| Fragrance | 0.20 |
| Water | q.s. for 100 |

2. Composition according to Claim 1, in which the amino-substituted 2-hydroxybenzophenone derivative is present in proportions ranging from 0.1% to 15% by weight.

3. Composition according to Claim 2, in which the amino-substituted 2-hydroxybenzophenone derivative is present in proportions ranging from 1% to 10% by weight.

4. Composition according to Claim 3, in which the amino-substituted 2-hydroxybenzophenone derivative is present in proportions ranging from 2% to 8% by weight, with respect to the total weight of the composition.

5. Composition according to any one of Claims 1 to 4, where the dibenzoylmethane derivative is present at contents ranging from 0.5% to 15% by weight, with respect to the total weight of the composition.

6. Composition according to Claim 5 where the dibenzoylmethane derivative is present at contents ranging from 1% to 10% by weight, with respect to the total weight of the composition.

7. Composition according to any one of Claims 1 to 6, **characterized in that** it additionally comprises other organic screening agents active in the UV-A and/or UV-B region(s).

8. Composition according to Claim 7, where the additional organic UV screening agent or agents are chosen from anthranilates; cinnamic derivatives; salicylic derivatives; camphor derivatives other than p-methylbenzylidene camphor; benzophenone derivatives other than those of formula (I); β,β-diphenylacrylate derivatives; benzotriazole derivatives; benzalmalonate derivatives; benzimidazole derivatives; imadazolines; bis-benzazolyl derivatives; p-aminobenzoic acid (PABA) derivatives; methylenebis(hydroxyphenylbenzotriazole) derivatives; screening polymers and screening silicones; dimers derived from α-alkylstyrene; or 4,4-diarylbutadienes.

9. Composition according to Claim 8, **characterized in that** the organic UV screening agent or agents are chosen from the following compounds:
- Ethylhexyl Salicylate,
- Ethylhexyl Methoxycinnamate,
- Octocrylene,
- Phenylbenzimidazole Sulfonic Acid,
- Terephthalylidene Dicamphor Sulfonic Acid,
- Benzophenone-3,
- Benzophenone-4,
- Benzophenone-5,
- 4-Methylbenzylidene Camphor,
- Disodium Phenyl Dibenzimidazole Tetrasulfonate,
- Anisotriazine,
- Ethylhexyl Triazone,
- Diethylhexyl Butamido Triazone,
- 2,4,6-tris(diisobutyl 4'-aminobenzalmalonate)-s-triazine,
- Methylene Bis-Benzotriazolyl Tetramethylbutylphenol,
- Drometrizole Trisiloxane,
and their mixtures,

10. Composition according to any one of Claims 1 to 9, **characterized in that** it additionally comprises coated or non-coated metal oxide pigments or nanopigments.

11. Composition according to Claim 10, **characterized in that** the said pigments or nanopigments are chosen from coated or non-coated titanium, zinc, iron, zirconium and cerium oxides and their mixtures.

12. Composition according to any one of Claims 1 to 11, **characterized in that** it additionally comprises at least one agent for the artificial tanning and/or browning of the skin.

13. Composition according to any one of Claims 1 to 12, **characterized in that** it additionally comprises at least one adjuvant chosen from fatty substances, organic solvents, ionic or non-ionic thickeners, demulcents, antioxidants, agents for combating free radicals, opacifying agents, stabilizing agents, emollients, silicones, α-hydroxy acids, antifoaming agents, moisturizing agents, vitamins, insect repellents, fragrances, preservatives, surfactants, anti-inflammatories, substance P antagonists, fillers, polymers, propellants, basifying or acidifying agents, or colourants.

14. Composition according to any one of Claims 1 to 13, **characterized in that** it is a composition which protects the human epidermis or an anti-sun composition and that it is provided in the form of a non-ionic vesicular dispersion, of an emulsion, in particular of an emulsion of oil-in-water type, of a cream, of a milk, of a gel, of a cream gel, of a suspension, of a dispersion, of a powder, of a solid stick, of a foam or of a spray.

15. Composition according to any one of Claims 1 to 13, **characterized in that** it is a composition for making up the eyelashes, eyebrows or skin and **in that** it is provided in the solid or pasty, anhydrous or aqueous, form of an emulsion, of a suspension or of a dispersion.

16. Composition according to any one of Claims 1 to 13, **characterized in that** it is a composition intended for the protection of the hair against ultraviolet rays and that it is provided in the form of a shampoo, of a lotion, of a gel, of an emulsion or of a non-ionic vesicular dispersion.

17. Method for improving the stability with respect to UV radiation of at least one dibenzoylmethane derivative in a cosmetic or dermatological composition not comprising p-methylbenzylidene camphor which consists in combining, with the said dibenzoylmethane derivative, an effective amount of at least one amino-substituted 2-hydroxybenzophenone derivative as defined in any one of the preceding claims, the ratio by weight of the 2-hydroxybenzophenone derivative to the dibenzoylmethane derivative being greater than 1.

## Claims (Claims for the following Contracting State(s): DE)

1. Cosmetic or dermatological composition, for topical use, **characterized in that** it comprises, in a cosmetically acceptable vehicle, at least:
a) 4-(tert-butyl)-4'-methoxydibenzoylmethane and
b) n-hexyl 2-(4-diethylamino-2-hydroxybenzoylbenzoate;
the said composition not comprising p-methylbenzylidene camphor; the ratio by weight of the 2-hydroxybenzophenone derivative to the dibenzoylmethane derivative being greater than 1 and the dibenzoylmethane derivative being present at contents ranging from 2% to 15% by weight, with respect to the total weight of the composition; with the proviso that the composition is different from the following formulations in which the amounts are expressed as percentages by weight, with respect to the total weight of the composition:
| **Ingredients** | **Composition 6** |
|---|---|
| Cetyl dimethicone copolyol | 0.5 |
| Polyglycerol polyricinoleate | 5.0 |
| n-Hexyl 2-(4-diethylamino-2-hydroxybenzoyl)benzoate | 5.0 |
| Phenylbenzimidazole sulfonic acid | 2.5 |
| Octocrylene | 5.0 |
| Dimethicone-diethyl benzalmalonate | 4.0 |
| Homosalate | 4.0 |
| Butyl methoxydibenzoylmethane | 3.0 |
| Micronized zinc oxide | 5.0 |
| Coco-caprylate/caprate | 10.0 |
| Dimethicone | 2.5 |
| Polydecene | 7.0 |
| Polyisobutene | 2.0 |
| Sodium chloride | 0.45 |
| Fragrance | 0.15 |
| Glycine soja | 2.0 |
| Phenoxyethanol | 1.0 |
| Water | q.s. for 100 |
| **Ingredients** | **Composition 9** |
|---|---|
| Self-emulsifying glycerol monostearate | 3.00 |
| Polyoxyethylene (30) cetyl stearyl ether | 1.00 |
| Stearyl alcohol | 3.00 |
| Butyl methoxydibenzoylmethane | 2.00 |
| Ethylhexyl triazone | 3.00 |
| n-Hexyl 2-(4-diethylamino-2-hydroxybenzoyl)benzoate | 3.50 |
| Phenylene-1,4-bis(2-benzimidazolyl-5,7-disulfonic acid, monosodium salt) | 0.5 |
| Dioctyl oxide | 3.50 |
| Dicaprylyl carbonate | 6.00 |
| Dimethicone polydimethylsiloxane | 1.00 |
| Glycerin | 5.00 |
| Tocopheryl acetate | 0.25 |
| Dioic acid | 0.20 |
| Diethylhexyl 2,6-naphthalate | 2.00 |
| alpha-Glucosylrutin | 0.20 |
| Paraben | 0.50 |
| Konkaben LMB® | 0.40 |
| Ethanol | 1.50 |
| Fragrance | q.s. |
| Water | q.s. for 100 |
| **Ingredients** | **Composition 12** |
|---|---|
| PEG-30 dipolyhydroxystearate | 5.00 |
| Butyl methoxydibenzoylmethane | 2.00 |
| Ethylhexyl triazone | 3.00 |
| n-Hexyl 2-(4-diethylamino-2-hydroxybenzoyl)benzoate | 4.50 |
| Octocrylene | 4.00 |
| Phenylene-1,4-bis(2-benzimidazolyl-5,7-disulfonic acid, monosodium salt) | 0.50 |
| Dimethicone diethyl benzalmalonate | 5.50 |
| Titanium dioxide | 1.50 |
| Zinc oxide | 2.00 |
| Liquid paraffin | 10.0 |
| Esters of caprylic and capric acids and of butylene glycol | 2.00 |
| Dicaprylyl carbonate | 6.00 |
| Dimethicone polydimethylsiloxane | 1.00 |
| Shea butter | 3.00 |
| Diethylhexyl 2,6-naphthalate | 6.50 |
| Octoxyglycerin | 1.00 |
| Glycine soja | 1.50 |
| Magnesium chloride | 1.00 |
| Tocopheryl acetate | 0.25 |
| Dioic acid | 0.50 |
| Paraben | 0.50 |
| Konkaben LMB® | 0.40 |
| Ethanol | 1.50 |
| Fragrance | q.s. |
| Water | q.s. for 100 |
| **Ingredients** | **Composition 15** |
|---|---|
| Glyceryl monostearate SE | 1.50 |
| PEG-100 stearate | 2.00 |
| Cetearyl sulfate | 0.75 |
| Stearyl alcohol | 2.00 |
| n-Hexyl 2-(4-diethylamino-2-hydroxybenzoyl)benzoate | 4.50 |
| Ethylhexyl triazone | 2.00 |
| Drometrizole trisiloxane | 1.00 |
| Butyl methoxydibenzoylmethane | 2.00 |
| Terephthalylidene dicamphor sulfonic acid | 0.50 |
| Zinc oxide HP1® | 2.00 |
| C₁₂-C₁₅ Alkyl benzoate | 7.00 |
| Dicaprylyl carbonate | 2.00 |
| Glycine soja | 1.50 |
| Phenoxyethanol | 0.40 |
| Ethanol | 4.50 |
| Fragrance | 0.40 |
| Water | q.s. for 100 |
| **Ingredients** | **Composition 24** |
|---|---|
| Glyceryl monostearate SE | 1.50 |
| PEG-40 stearate | 2.00 |
| Cetearyl sulfate | 0.75 |
| Stearyl alcohol | 2.00 |
| n-Hexyl 2-(4-diethylamino-2-hydroxybenzoyl)benzoate | 4.50 |
| Butyl methoxydibenzoylmethane | 2.00 |
| Ethylhexyl triazone | 2.00 |
| Drometrizole trisiloxane | 1.00 |
| Terephthalylidene dicamphor sulfonic acid | 0.50 |
| Diethylhexyl 2,6-naphthalate | 5.50 |
| Zinc oxide HP1® | 2.00 |
| C₁₂-C₁₅ Alkyl benzoate | 7.00 |
| Dicaprylyl carbonate | 2.00 |
| Glycine soja | 1.50 |
| Phenoxyethanol | 0.40 |
| Ethanol | 4.50 |
| Fragrance | 0.40 |
| Water | q.s. for 100 |
| **Ingredients** | **Composition 37** |
|---|---|
| Glyceryl stearate citrate | 1.00 |
| PEG-100 stearate | 2.00 |
| Cetyl phosphate | 0.75 |
| Stearyl alcohol | 2.00 |
| Dermacryl 79® | 2.00 |
| n-Hexyl 2-(4-diethylamino-2-hydroxybenzoyl)benzoate | 4.50 |
| Butyl methoxydibenzoylmethane | 2.00 |
| Ethylhexyl triazone | 2.00 |
| Drometrizole trisiloxane | 1.00 |
| Terephthalylidene dicamphor sulfonic acid | 0.50 |
| Diethylhexyl 2,6-naphthalate | 10.00 |
| Zinc oxide HP1® | 2.00 |
| C₁₂-C₁₅ Alkyl benzoate | 7.00 |
| Dicaprylyl carbonate | 2.00 |
| Glycine soja | 1.50 |
| Phenoxyethanol | 0.40 |
| Ethanol | 4.50 |
| Fragrance | 0.40 |
| Water | q.s. for 100 |
| **Ingredients** | **Composition 44** |
|---|---|
| Glyceryl monostearate SE | 0.50 |
| Glyceryl stearate citrate | 2.00 |
| PEG-40 stearate | 0.50 |
| n-Hexyl 2-(4-diethylamino-2-hydroxybenzoyl)benzoate | 4.00 |
| Butyl methoxydibenzoylmethane | 2.00 |
| Ethylhexyl triazone | 4.00 |
| Parsol SLX® | 3.50 |
| Mexoryl SX® | 0.25 |
| Bisimidazylate | 1.00 |
| Phenylbenzimidazole sulfonic acid | 0.50 |
| Titanium dioxide MT100 TV® | 1.00 |
| Esters of caprylic and capric acids and of butylene glycol | 5.00 |
| Cyclomethicone | 2.00 |
| PVP/eicosene copolymer | 0.50 |
| Glycerin | 3.00 |
| Propylene glycol | 1.50 |
| Xanthan gum | 0.15 |
| Vitamin E acetate | 0.50 |
| alpha-Glucosylrutin | 0.35 |
| Glycine soja | 1.00 |
| Pentasodium ethylenediaminetetramethylenephosphonate (PETP) | 0.25 |
| Trisodium EDTA | 0.10 |
| Methylparaben | 0.15 |
| Phenoxyethanol | 1.00 |
| Fragrance | 0.20 |
| Water | q.s. for 100 |
| **Ingredients** | **Composition 47** |
|---|---|
| Lauryl dimethicone copolyol | 1.00 |
| PEG-30 dipolyhydroxystearate | 5.00 |
| n-Hexyl 2-(4-diethylamino-2-hydroxybenzoyl)benzoate | 5.00 |
| Methylene bis-benzotriazolyl tetramethylbutylphenol | 2.00 |
| Butyl methoxydibenzoylmethane | 2.00 |
| Ethylhexyl triazone | 3.00 |
| Parsol SLX® | 3.50 |
| octocrylene | 8.00 |
| Bisimidazylate | 0.50 |
| Titanium dioxide MT-100 Z | 3.00 |
| Zinc oxide Z-cote HP1® | 6.00 |
| Mineral oil | 10.00 |
| Esters of caprylic and capric acids and of butylene glycol | 2.00 |
| Cyclomethicon | 5.00 |
| Pentasodium ethylenediaminetetramethylenephosphonate (PETP) | 0.25 |
| Ethylhexylglycerin | 1.00 |
| Glycine soja | 1.50 |
| Butylene glycol | 10.00 |
| MgCl₂ | 1.00 |
| Vitamin E | 0.25 |
| Ethanol | 4.50 |
| Fragrance | 0.20 |
| Water | q.s. for 100 |
| **Ingredients** | **Composition 51** |
|---|---|
| PEG-30 dipolyhydroxystearate | 5.00 |
| Dimethicone/vinyl dimethicone crosspolymer | 5.00 |
| n-Hexyl 2-(4-diethylamino-2-hydroxybenzoyl)benzoate | 3.00 |
| Methylene bis-benzotriazolyl tetramethylbutylphenol | 2.00 |
| Butyl methoxydibenzoylmethane | 2.00 |
| Ethylhexyl triazone | 3.00 |
| Parsol SLX® | 3.50 |
| Octocrylene | 8.00 |
| Bisimidazylate | 0.50 |
| Titanium dioxide MT-100 Z® | 3.00 |
| Zinc oxide Z-cote HP1® | 6.00 |
| Mineral oil | 10.00 |
| Esters of caprylic and capric acids and of butylene glycol | 2.00 |
| Dimethicone | 12.50 |
| Diethylhexyl 2,6-naphthalate | 5.50 |
| Ethylhexylglycerin | 1.00 |
| Butylene glycol | 10.00 |
| Glycine soja | 1.50 |
| MgCl₂ | 1.00 |
| Vitamin E | 0.25 |
| Ethanol | 4.50 |
| Fragrance | 0.20 |
| Water | q.s. for 100 |
| **Ingredients** | **Composition 53** |
|---|---|
| Glyceryl monostearate SE | 0.50 |
| Glyceryl stearate citrate | 2.00 |
| PEG-40 stearate | 0.50 |
| Vinyl dimethicone/methicone silsesquioxane crosspolymer (KSP-100 Shin-Etsu) | 10.00 |
| n-Hexyl 2-(4-diethylamino-2-hydroxybenzoyl)benzoate | 4.00 |
| Butyl methoxydibenzoylmethane | 2.00 |
| Ethylhexyl triazone | 4.00 |
| Parsol SLX® | 3.50 |
| Mexoryl SX® | 0.25 |
| Phenyl dibenzimidazole tetrasulfonic acid | 1.00 |
| Phenylbenzimidazole sulfonic acid | 0.50 |
| Titanium dioxide MT 100 TV® | 1.00 |
| Esters of caprylic and capric acids and of butylene glycol | 5.00 |
| Cyclomethicone | 2.00 |
| PVP/hexadecene copolymer | 0.50 |
| Glycerin | 3.00 |
| Xanthan gum | 0.15 |
| Vitamin E acetate | 0.50 |
| alpha-Glucosylrutin | 0.35 |
| Diethylhexyl 2,6-naphthalate | 4.00 |
| Trisodium EDTA | 0.10 |
| Methylparaben | 0.15 |
| Phenoxyethanol | 1.00 |
| Fragrance | 0.20 |
| Water | q.s. for 100 |
| **Ingredients** | **Composition 56** |
|---|---|
| PEG-30 dipolyhydroxystearate | 5.00 |
| Vinyl dimethicone/methicone silsesquioxane crosspolymer modified by fluoroalkyl groups (KSP-200 Shin-Etsu) | 2.75 |
| n-Hexyl 2-(4-diethylamino-2-hydroxybenzoyl)benzoate | 3.00 |
| Methylene bis-benzotriazolyl tetramethylbutylphenol | 2.00 |
| Butyl methoxydibenzoylmethane | 2.00 |
| Ethylhexyl triazone | 3.00 |
| Parasol SLX® | 3.50 |
| Phenyl dibenzimidazole tetrasulfonic acid | 0.50 |
| Titanium dioxide MT 100 Z® | 3.00 |
| Zinc oxide Z-cote HP1® | 6.00 |
| Mineral oil | 10.00 |
| Esters of caprylic and capric acids and of butylene glycol | 2.00 |
| Dimethicone | 12.50 |
| Diethylhexyl 2,6-naphthalate | 5.50 |
| Ethylhexylglycerin | 1.00 |
| Butylene glycol | 10.00 |
| Glycine soja | 1.50 |
| MgCl₂ | 1.00 |
| Vitamin E | 0.25 |
| Ethanol | 4.50 |
| Fragrance | 0.20 |
| Water | q.s. for 100 |

2. Composition according to Claim 1, in which the amino-substituted 2-hydroxybenzophenone derivative is present in proportions ranging from 0.1% to 15% by weight.

3. Composition according to Claim 2, in which the amino-substituted 2-hydroxybenzophenone derivative is present in proportions ranging from 1% to 10% by weight.

4. Composition according to Claim 3, in which the amino-substituted 2-hydroxybenzophenone derivative is present in proportions ranging from 2% to 8% by weight, with respect to the total weight of the composition.

5. Composition according to any one of Claims 1 to 4, where the dibenzoylmethane derivative is present at contents ranging from 0.5% to 15% by weight, with respect to the total weight of the composition.

6. Composition according to Claim 5, where the dibenzoylmethane derivative is present at contents ranging from 1% to 10% by weight, with respect to the total weight of the composition.

7. Composition according to any one of Claims 1 to 6, **characterized in that** it additionally comprises other organic screening agents active in the UV-A and/or UV-B region(s).

8. Composition according to Claim 7, where the additional organic UV screening agent or agents are chosen from anthranilates; cinnamic derivatives; salicylic derivatives; camphor derivatives other than p-methylbenzylidene camphor; benzophenone derivatives other than those of formula (I); β,β-diphenylacrylate derivatives; benzotriazole derivatives; benzalmalonate derivatives; benzimidazole derivatives; imadazolines; bis-benzazolyl derivatives; p-aminobenzoic acid (PABA) derivatives; methylenebis(hydroxyphenylbenzotriazole) derivatives; screening polymers and screening silicones; dimers derived from α-alkylstyrene; or 4,4-diarylbutadienes.

9. Composition according to Claim 8, **characterized in that** the organic UV screening agent or agents are chosen from the following compounds:
- Ethylhexyl Salicylate,
- Ethylhexyl Methoxycinnamate,
- Octocrylene,
- Phenylbenzimidazole Sulfonic Acid,
- Terephthalylidene Dicamphor Sulfonic Acid,
- Benzophenone-3,
- Benzophenone-4,
- Benzophenone-5,
- 4-Methylbenzylidene Camphor,
- Disodium Phenyl Dibenzimidazole Tetrasulfonate,
- Anisotriazine,
- Ethylhexyl Triazone,
- Diethylhexyl Butamido Triazone,
- 2,4,6-tris(diisobutyl 4'-aminobenzalmalonate)-s-triazine,
- Methylene Bis-Benzotriazolyl Tetramethylbutylphenol,
- Drometrizole Trisiloxane,
and their mixtures.

10. Composition according to any one of Claims 1 to 9, **characterized in that** it additionally comprises coated or non-coated metal oxide pigments or nanopigments.

11. Composition according to Claim 10, **characterized in that** the said pigments or nanopigments are chosen from coated or non-coated titanium, zinc, iron, zirconium and cerium oxides and their mixtures.

12. Composition according to any one of Claims 1 to 11, **characterized in that** it additionally comprises at least one agent for the artificial tanning and/or browning of the skin.

13. Composition according to any one of Claims 1 to 12, **characterized in that** it additionally comprises at least one adjuvant chosen from fatty substances, organic solvents, ionic or non-ionic thickeners, demulcents, antioxidants, agents for combating free radicals, opacifying agents, stabilizing agents, emollients, silicones, α-hydroxy acids, antifoaming agents, moisturizing agents, vitamins, insect repellents, fragrances, preservatives, surfactants, anti-inflammatories, substance P antagonists, fillers, polymers, propellants, basifying or acidifying agents, or colourants.

14. Composition according to any one of Claims 1 to 13, **characterized in that** it is a composition which protects the human epidermis or an anti-sun composition and that it is provided in the form of a non-ionic vesicular dispersion, of an emulsion, in particular of an emulsion of oil-in-water type, of a cream, of a milk, of a gel, of a cream gel, of a suspension, of a dispersion, of a powder, of a solid stick, of a foam or of a spray.

15. Composition according to any one of Claims 1 to 13, **characterized in that** it is a composition for making up the eyelashes, eyebrows or skin and **in that** it is provided in the solid or pasty, anhydrous or aqueous, form of an emulsion, of a suspension or of a dispersion.

16. Composition according to any one of Claims 1 to 13, **characterized in that** it is a composition intended for the protection of the hair against ultraviolet rays and that it is provided in the form of a shampoo, of a lotion, of a gel, of an emulsion or of a non-ionic vesicular dispersion.

17. Method for improving the stability with respect to UV radiation of at least one dibenzoylmethane derivative in a cosmetic or dermatological composition not comprising p-methylbenzylidene camphor which consists in combining, with the said dibenzoylmethane derivative, an effective amount of at least one amino-substituted 2-hydroxybenzo-phenone derivative as defined in any one of the preceding claims, the ratio by weight of the 2-hydroxybenzophenone derivative to the dibenzoylmethane derivative being greater than 1.
